# EUROPÄISCHE PATENTSCHRIFT

(11) **EP 2 349 128 B1**
(45) Veröffentlichungstag und Bekanntmachung des Hinweises auf die Patenterteilung: **31.08.2016**
(21) Anmeldenummer: 09743872.5
(22) Anmeldetag: 27.10.2009
(51) Int. Cl.: A61F 2/90, D04C 1/06

(54) **MEDIZINISCHES IMPLANTAT UND VERFAHREN ZUM HERSTELLEN EINES IMPLANTATS**
MEDICAL IMPLANT AND METHOD FOR PRODUCING AN IMPLANT
IMPLANT MÉDICAL ET PROCÉDÉ DE FABRICATION D'UN IMPLANT

(30) Priorität: 29.10.2008 DE 102008053659; 27.01.2009 DE 102009006180
(43) Veröffentlichungstag der Anmeldung: 03.08.2011
(73) Patentinhaber: Acandis GmbH & Co. KG, 76327 Pfinztal (DE)
(72) Erfinder: CATTANEO, Giorgio, 76199 Karlsruhe (DE); BAIDINGER, Otto, 75175 Pforzheim (DE)
(74) Vertreter: Kilchert, Jochen
(86) Internationale Anmeldenummer: PCT/EP2009/007675
(87) Internationale Veröffentlichungsnummer: WO 2010/049123

(56) Entgegenhaltungen:
- DE-A1- 10 127 602
- DE-A1-102007 012 964
- US-A1- 2006 116 752
- US-A1- 2006 271 166

## Beschreibung

Die Erfindung betrifft ein medizinisches Implantat sowie ein Verfahren zur Herstellung eines derartigen Implantats. Ein Implantat mit den Merkmalen des Oberbegriffs des Anspruchs 1 ist aus DE 10 2007 012 964 A1 bekannt.

Geflochtene Implantate wie beispielsweise geflochtene Stents, haben den Vorteil, dass diese flexibel und sehr feinmaschig ausgebildet sein können. Dadurch wird die Strömungsbeeinflussung bei der Behandlung von Aneurysmen und die Filterfunktion bei der Ausweitung von Stenosen verbessert. Überdies weisen geflochtene Implantate geschlossene Zellen auf, die eine Wiederpositionierung der Implantate vereinfachen, da diese in ein Zufuhrsystem, bspw. einen Katheter wieder eingezogen werden können.

Es ist bekannt, Stents aus einem einzigen Draht zu bilden, der mehrfach die Richtung wechselt und dabei an den Enden jeweils eine Schlaufe bildet. Die Verwendung eines einzigen Drahtes zur Herstellung eines Stents hat den Nachteil, dass der gesamte Stent auf eine Drahtstärke festgelegt ist. Ein weiterer Nachteil eines eindrahtigen Geflechtes besteht darin, dass die Feinmaschigkeit des Stents limitiert ist. Hinzu kommt, dass die Anzahl der Drahtüberkreuzungen bei der Herstellung aus einem einzigen Draht beschränkt ist.

Demgegenüber haben geflochtene Stents, die beispielsweise mit Hilfe von Textilmaschinen aus mehreren Drähten hergestellt werden, den Vorteil, dass unterschiedliche Wandstärken innerhalb desselben Stents ausgebildet werden können. Die Drähte mit größerem Durchmesser verleihen dem Stent Kraft in radialer Richtung. Feinere Drähte führen zu einer feinmaschigen Struktur. Die Herstellung eines geflochtenen Stents aus mehreren Einzeldrähten hat ferner den Vorteil, dass Drähte aus unterschiedlichen Materialien verwendet werden können, wobei einige Materialien, wie beispielsweise Nitinol, Kobalt-Legierungen, Edelstahl, dem Stent die notwendige Kraft und andere Materialien, wie beispielsweise Platin, Tantal, die Röntgensichtbarkeit verleihen. Besonders vorteilhaft ist die hohe Anzahl an Drähten und daher die Feinmaschigkeit. Auch die Herstellung kann besser automatisiert werden.

Geflochtene Stents sind beispielsweise aus DE 101 27 602 A1 und US 2006/116752 A1 bekannt.

Zur Herstellung eines geflochtenen Stents wird üblicherweise ein längliches Rundgeflecht gebildet, das an den axialen Enden geschnitten bzw. abgelängt wird. Dadurch entstehen an den axialen Rändern des Stents freie Drahtenden, was zu verschiedenen Nachteilen führt:

Die Drähte können sich voneinander lösen und somit den jeweiligen Überkreuzungsbereich im Geflecht verlassen. Die losen Drähte können keine oder nur eine reduzierte Radialkraft ausüben, wodurch die Funktionalität der Stents bei der Behandlung von Aneurysmen und Stenosen beeinträchtigt wird. Die freien Enden führen zu einer Verletzungsgefahr bzw. zu einer Gefahr von Entzündungen und zur Bildung von Stenosen, da diese die Gefäßwand durchstechen können. Weitere Nachteile geflochtener Stents mit offenen Enden treten in Zusammenwirken mit einem Zufuhrsystem, beispielsweise einem Katheter, auf. So können sich beispielsweise die freien Enden beim Crimpen im Zufuhrsystem verfangen mit der Folge, dass sich der Stent nicht öffnet. Dadurch besteht eine Gerinnungsgefahr und die Funktionalität des Stents wird beeinträchtigt. Wenn der Stent aus dem Zufuhrsystem geschoben wird, z.B. bei der Entlassung durch einen Stabilizer, wird der Stent mit einer Druckkraft beaufschlagt, wodurch die losen Drähte stark gestaucht werden, da eine stabile Struktur zumindest im Randbereich des Stents fehlt. Dabei können die Drähte beschädigt und verbogen werden. Dies führt zu einer erhöhten Verletzungsgefahr insbesondere dann, wenn die freien Drahtenden radial nach außen gebogen sind.

Selbst wenn es beim Setzen der Stents nicht zu Beschädigungen oder Stauchungen kommt, können die freien Drahtenden zu einer Beeinträchtigung der Funktionalität führen, da die losen Drähte im Bereich der axialen Stentenden aufgrund der relativ instabilen Struktur nicht die erforderliche Radialkraft besitzen, um sich nach Entlassung aus dem Zufuhrsystem radial nach außen aufzuweiten. Vielmehr ragen die losen Drähte in die Blutbahn hinein und erhöhen dadurch die Gerinnungsgefahr.

US 4,655,771 begegnet dem Problem der losen Drahtenden dadurch, dass diese durch U-förmige Verbindungsglieder verbunden sind, wodurch allerdings Spannungen im Stent induziert werden, die zu einer Deformierung des Stents führen können. Im Übrigen ist die Verbindung einzelner Drähte durch U-förmige Verbindungsmittel bei sehr feinmaschigen Stents mit einer Vielzahl von Einzeldrähten herstellungstechnisch sehr schwer bis praktisch nicht realisierbar.

US 5,061,275 offenbart einen Stent, dessen lose Enden durch eine Laserbehandlung rundgeschmolzen sind, um so einer Traumatisierung entgegen zu wirken. Die Stabilität des Stents wird dabei durch lokale plastische Verformungen der einzelnen Drähte im Bereich der Überkreuzungen bzw. Knoten des Geflechts verbessert, was herstellungstechnisch aufwändig ist. Im Übrigen nimmt bei diesem Stent die Radialkraft im Bereich der losen Enden ab, so dass die Gefahr besteht, dass diese in die Blutbahn hineinragen.

Die eingangs genannte DE 10 2007 012 967 A1 offenbart ein Implantat, beispielsweise einen Inliner oder Flow-Diverter, der als Rundgeflecht ausgebildet ist und bei dem die an den Implantatenden herausstehenden einzelnen Filamentenden zumindest paarweise zusammengeführt und miteinander verbunden sind. Die verbundenen Filamentenden werden atraumatisch umgeformt, so dass die Verletzungsgefahr verringert wird. Die Stabilität und die atraumatischen Eigenschaften der Implantatenden werden hierdurch nicht ausreichend verbessert, da die gebündelten Filamentenden beim Entlassen des Implantats aus einem Zufuhrsystem verbogen werden können.

Der Erfindung liegt die Aufgabe zu Grunde, das Implantat der eingangs genannten Art so zu verbessern, dass eine verbesserte Stabilität im Bereich der Implantatenden erreicht und die Gefahr einer Beeinträchtigung der Funktionalität des Implantats verringert wird. Der Erfindung liegt ferner die Aufgabe zu Grunde, ein Verfahren zur Herstellung eines derartigen medizinischen Implantats anzugeben.

Erfindungsgemäß wird diese Aufgabe im Hinblick auf das medizinische Implantat durch den Gegenstand des Anspruchs 1 gelöst. Im Hinblick auf das Verfahren wird die Aufgabe durch den Gegenstand des Anspruchs 13 gelöst.

Die Erfindung beruht auf dem Gedanken, ein medizinisches Implantat, insbesondere einen Stent, mit einer aus mehreren Drähten geflochtenen Wandung anzugeben, die sich entlang einer Längsachse erstreckt und zumindest abschnittsweise um die Längsachse gekrümmt ist. Jeweils wenigstens zwei Drahtenden der Drähte sind zu wenigstens zwei ersten Geflechtenden verbunden, die einen um die Längsachse erstreckten ersten Umfangsabschnitt der Wandung bilden. Jeweils mindestens zwei erste Geflechtenden sind zu einem oder mehreren zweiten Geflechtenden verbunden, wobei die zweiten Geflechtenden einen um die Längsachse erstreckten zweiten Umfangsabschnitt der Wandung bilden, der dem ersten Umfangsabschnitt in längsaxialer Richtung nachgeordnet ist. Alternativ sind die zweiten Geflechtenden in Umfangsrichtung der Wandung angeordnet.

Die Erfindung geht über die aus DE 10 2007 012 964 A1 bekannte Implantatstruktur hinaus, bei der die einzelnen Filamentenden bzw. Drahtenden zu einem ersten Geflechtende verbunden sind, das frei in Axialrichtung über den Rand des Implantats vorsteht und dementsprechend empfindlich ist (Fig. 4 der DE 10 2007 012 964 A1). Im Rahmen der Erfindung hingegen wirken mehrere, d.h. mindestens zwei aus einzelnen Filamentenden gebildete erste Geflechtenden zusammen. Konkret sind mindestens zwei erste Geflechtenden zu einem oder mehreren zweiten Geflechtenden verbunden.

Durch die Verbindung wenigstens zweier erster Geflechtenden zu einem oder zu mehreren zweiten Geflechtenden, wird die Stabilität des Implantats an den axialen Enden, sowie die Radialkraft im Bereich der axialen Enden signifikant erhöht. Dies hat den Vorteil, dass sich die Drähte radial nach außen ausweiten, wodurch vermieden wird, dass sich Elemente des Implantats nach innen in die Blutbahn bewegen. Ferner wird vermieden, dass sich die ersten Geflechtenden ineinander verhaken. Durch die Verbindung der ersten Geflechtenden zu zweiten Geflechtenden wird eine erhöhte Fixierung der Geflechtenden erreicht. Die axialen Enden des Implantats sind atraumatisch.

Die erfindungsgemäße Anordnung der zweiten Geflechtenden, die einen um die Längsachse erstreckten zweiten Umfangsabschnitt der Wandung bilden, der dem ersten Umfangsabschnitt in längsaxialer Richtung nachgeordnet ist, hat den Vorteil, dass das erfindungsgemäße Prinzip der Interaktion mehrerer Geflechtenden in Längserstreckung des Implantats wiederholt werden kann, wodurch eine weitere Verbesserung in der Stabilität im Bereich der axialen Implantatenden erreicht wird.

Bei der erfindungsgemäßen Alternative, wonach die zweiten Geflechtenden in Umfangsrichtung der Wandung angeordnet sind, wird der Vorteil erreicht, dass ein an den Rändern im Wesentlichen geschlossenes Implantat gebildet wird, bei dem ein Vorstehen von Implantatelementen in Axialrichtung vermieden wird, da diese im Bereich der Implantatenden bzw. -ränder in Umfangsrichtung der Wandung ausgerichtet sind. Dies hat den Vorteil, dass keine exponierten Implantat-Elemente beim Entlassen des Implantats aus einem Zuführsystem verbogen werden können oder sich verhaken. Es ist möglich, dass die zweiten Geflechtenden teilweise in Umfangsrichtung, oder zumindest vorwiegend in Umfangsrichtung angeordnet sind. Eine axiale Komponente des Verlaufs des Geflechtendes ist möglich. Die tangentiale Komponente ist größer als die axiale Komponente. Zumindest teilweise in Umfangsrichtung ausgerichtete Geflechtenden sind atraumatischer als rein axial ausgerichtete Geflechtenden.

Unabhängig von der beanspruchten Erfindung wird ein medizinisches Implantat, insbesondere eineStent, mit einer aus mehreren Drähten geflochtenen Wandung offenbart, die sich entlang einer Längsachse erstreckt und zumindest abschnittsweise um die Längsachse gekrümmt ist, wobei wenigstens zwei Drahtenden der Drähte zu wenigstens einem Geflechtende verbunden sind, das in einem um die Längsachse erstreckten ersten Umfangsabschnitt der Wandung angeordnet ist. Die Drähte, deren Drahtenden zur Bildung desselben Geflechtendes miteinander verbunden sind, weisen dieselbe Flechtrichtung auf. Das Geflechtende ist zumindest teilweise in Umfangsrichtung der Wandung angeordnet. Das Geflechtende kann eine axiale Komponente aufweisen. Das Geflechtende kann ganz in Umfangsrichtung erstreckt sein und nur eine tangentiale Ausrichtung aufweisen.

Durch die Verbindung von Drahtenden bzw. Drähten mit derselben Flechtrichtung, d.h. nur im Uhrzeigersinn oder nur entgegen dem Uhrzeigersinn, zu einem Geflechtende wird erreicht, dass die Drähte sanft in das Geflechtende hineinlaufen und ohne signifikante plastische Verformung miteinander verbunden sind. Da die Drähte bzw. Drahtenden tangential zueinander zusammenlaufen, wird eine abrupte Winkeländerung im Bereich der Verbindung vermieden. Beim Komprimieren in kleinen Zufuhrsystemen erfolgt keine wesentliche Verformung bzw. Beschädigung der Drähte. Die Ausrichtung des Geflechtendes in Umfangsrichtung der Wandung hat den Vorteil, dass sich das Implantatende gut an die Gefäßwand anlegt, wenn das Geflecht aus dem Einführungskatheter entlassen wird. Dabei hält die Krümmung des Gefäßes das Implantatende stabil, das radial nicht nach innen in die Blutbahn hineinragt. Das auf dem Gefäßradius verlaufende Implantatende ist gut stabilisiert und stellt daher eine verringerte Verletzungsgefahr dar.

Bei einer bevorzugten Ausführungsform der Erfindung ist vorgesehen, dass die das axiale Implantatende, insbesondere das axiale Stentende bildenden Geflechtenden, insbesondere die zweiten Geflechtenden, schlaufenförmige Enden mit einem in distaler Richtung erstreckten ersten Abschnitt, einem gekrümmten zweiten Abschnitt und einem in proximaler Richtung erstreckten dritten Abschnitt umfassen, die in Umfangsrichtung verteilt, im Wesentlichen sich axial erstreckende erste Geflechtenden verbinden, wobei wenigstens zwei in Umfangsrichtung unmittelbar angrenzend angeordnete schlaufenförmige Enden miteinander verbunden sind.

Die miteinander verbundenen und sich gegenseitig abstützenden Schlaufen erhöhen die Gesamtstabilität des Geflechtes, da aufgrund der Symmetrie der Kräfte ein lokales Kräftegleichgewicht herrscht. Das Kräftegleichgewicht im Bereich der Schlaufe bedeutet, dass die einzelnen Schlaufen fixiert sind und beim Verformen des Implantats nicht in eine Umfangsrichtung relativ zu einer anderen Schlaufe gedrückt werden können. Die Struktur des Implantats wird nicht verzerrt, da Nachbarschlaufen durch die gleiche Kraft stabilisiert werden.

Auch bei dieser Ausführungsform können mehr als zwei Umfangsabschnitte vorgesehen sein, so dass das erfindungsgemäße Prinzip, erste Geflechtenden zu zweiten Geflechtenden zu verbinden, mehrfach wiederholbar ist. Die das Implantatende bildenden atraumatischen schlaufenförmigen Enden begrenzen das Implantat in axialer Richtung und bilden den Abschluss der jeweils untereinander verbundenen, jeweils aus mehreren Drahtenden bestehenden Geflechtenden. Dabei bezeichnet allgemein der Begriff "erstes Geflechtende" ein in axialer Richtung einem weiteren Geflechtende, nämlich dem zweiten Geflechtende vorgeordnetes Geflechtende. Dies gilt für alle in der Anmeldung offenbarten Ausführungsformen. Die Erfindung und deren Ausführungsformen sind daher nicht auf ein Implantat mit nur zwei in axialer Richtung einander nachgeordneten Geflechtenden eingeschränkt.

Vorzugsweise umfassen die schlaufenförmigen Enden jeweils eine Gruppe der Drahtenden des ersten Geflechtendes, insbesondere wenigstens ein, zwei oder mehr als zwei Drahtenden, die mit der Gruppe der Drahtenden eines weiteren ersten Geflechtendes verbunden sind. Die Bildung einer Gruppe von Drahtenden, also die Verwendung einer Teilanzahl der Drahtenden eines ersten Geflechtendes zur Bildung der schlaufenförmigen Enden bietet die Voraussetzung für unterschiedlich konfigurierte Implantatenden, bei denen verschiedene Schlaufen sich gegenseitig stabilisierend kombinierbar sind.

Besonders bevorzugt ist eine Ausführungsform, bei der die Drahtenden des ersten Geflechtendes im Bereich des zweiten Umfangsabschnittes verzweigt sind derart, dass die Gruppen der Drahtenden wenigstens zwei schlaufenförmige Enden bilden, die in entgegengesetzten Umfangsrichtungen angeordnet sind, wobei die Drahtenden eines verzweigten ersten Geflechtendes mit den Drahtenden jeweils eines unverzweigten ersten Geflechtendes verbunden sind. Durch die Verzweigung der Drahtenden eines ersten Geflechtendes wird eine schirmartige Struktur erreicht, wobei die (bei der Herstellung des Implantats) freien Enden der verzweigten Drahtenden Anschlussmöglichkeiten für die Verbindung mit angrenzenden Schlaufen bzw. Drahtenden eröffnen. Aufgrund seiner hohen Symmetrie auch in den lokalen Verbindungsstellen zwischen den angrenzenden Gruppen bzw. Drahtenden zeichnet sich diese Ausführungsform durch ein besonders gutes Kräftegleichgewicht und somit durch eine besonders hohe Gesamtstabilität des Geflechtes aus.

Die Drahtenden des unverzweigten ersten Geflechtendes und die Drahtenden der zugehörigen schlaufenförmigen Enden können entgegengerichtet, insbesondere auf Stoß entgegengerichtet angeordnet sein. Auf diese Weise können die Verbindungsstellen zwischen den Gruppen, Schlaufen bzw. Drahtenden mit geringem Platzbedarf verwirklicht werden.

Die Drahtenden der miteinander verbundenen Gruppen können im Bereich der Verbindung parallel angeordnet sein, wodurch eine besonders gute Fixierung der schlaufenförmigen Enden und eine gute Kraftübertragung zwischen den schlaufenförmigen Enden erreicht wird.

Die Drahtenden des ersten Geflechtendes können untereinander und/oder mit Drahtenden wenigstens eines weiteren ersten Geflechtendes mechanisch, insbesondere durch Verdrillen und/oder Verflechten und/oder durch ein Verbindungselement und/oder stoffschlüssig, insbesondere durch Löten, Schweißen oder Kleben verbunden sein. Die durch Verdrillen erzielte mechanische Verbindung hat den Vorteil, dass diese einfach realisierbar und mit sehr geringem Platzbedarf verbunden ist. Dadurch sind Verbindungen im Zusammenhang mit sehr kleinen Verbindungselementen oder auch ohne Verbindungselemente und das Crimpen auf kleine Durchmesser möglich. Die durch Flechten herstellbaren mechanischen Verbindungen haben den Vorteil, dass die verschiedenen miteinander verbundenen Abschnitte gut stabilisiert sind. Es ist auch möglich, unterschiedliche mechanische Verbindungsarten miteinander zu kombinieren. Dasselbe gilt für die Kombination der vorstehend genannten mechanischen Verbindungen, nämlich Verdrillen oder Verflechten mit einem Verbindungselement. Zusätzlich können die Drahtenden bzw. die Gruppen der Drahtenden stoffschlüssig untereinander bzw. miteinander verbunden sein.

Bei einer weiteren Ausführungsform bilden die schlaufenförmigen Enden mehrere Ebenen derart, dass wenigstens ein schlaufenförmiges Ende mehrere erste Geflechtenden übergreift, die durch schlaufenförmige Enden untereinander verbunden sind. Dadurch wird die Gesamtstabilität des Implantats weiter verbessert. Die schlaufenförmigen Enden können überlappend angeordnet sein. Auch dies ist eine Möglichkeit, die Gesamtstabilität des Implantats im Bereich der axialen Enden zu erhöhen.

Bei einer weiteren besonders bevorzugten Ausführungsform sind mehr als zwei Umfangsabschnitte vorgesehen, die in längsaxialer Richtung nachgeordnet und jeweils durch miteinander verbundene weitere Geflechtenden gebildet sind. Die bei dieser Ausführungsform vorgenommene Wiederholung des erfindungsgemäßen Prinzips, wonach die durch Zusammenführen von Geflechtenden gebildeten weiteren Geflechtenden wiederum zu neuen Geflechtenden verbunden sind, erhöht weiter die Stabilität der beiden Implantatenden.

Die Drähte, deren Drahtenden zur Bildung jeweils eines Geflechtendes verbunden sind und/oder die Geflechtenden, die zur Bildung jeweils eines weiteren Geflechtendes verbunden sind, können gegenläufige Richtungen aufweisen. Das bedeutet, dass die miteinander verbundenen Drähte bzw. Geflechtenden in unterschiedlichen Spiralrichtungen, insbesondere im Fall eines Rundgeflechts, wie bei einem Stent, angeordnet sind. Diese Ausführungsform ist herstellungstechnisch günstig.

Die Geflechtenden können jeweils einen Verbindungsabschnitt aufweisen, wobei die Verbindungsabschnitte gegengerichtet angeordnet und miteinander verbunden sind. Dabei können die Verbindungsabschnitte mit ihren Stirnflächen in Gegenüberstellung angeordnet und/oder die Verbindungsabschnitte parallel überlappend angeordnet und/oder die Verbindungsabschnitte unter einem Winkel α miteinander verbunden sein. Durch die entgegengerichtete Anordnung der Verbindungsabschnitte können Abschlussverbindungen erreicht werden, die tangential in Umfangsrichtung ausgerichtet sind, wodurch auf einfache Weise atraumatische Implantatenden gebildet werden.

Alternativ ist es auch möglich, die Geflechtenden so auszubilden, dass diese jeweils einen Verbindungsabschnitt aufweisen, wobei die Verbindungsabschnitte gleichgerichtet angeordnet und miteinander verbunden sind. Die gleichgerichtete Anordnung der Verbindungsabschnitte bietet die Möglichkeit, die Geflechtenden zumindest teilweise in axialer Richtung des Implantats, d.h. mit wenigstens einer Axialkomponente auszurichten, so dass die derart ausgerichteten Geflechtenden mit anderen Geflechtenden verbunden werden können, die ebenfalls mit wenigstens einer Axialkomponente ausgerichtet sind. Diese Ausführungsform hat den Vorteil, dass auf einfache Weise die Verbindung verschiedener Geflechtenden mehrfach in axialer Richtung des Implantats wiederholt werden kann.

Bei einer weiteren Ausführungsform weisen die Drähte, die an Drahtenden zur Bildung jeweils eines ersten Geflechtendes verbunden sind, und/oder die Geflechtenden, die zur Bildung jeweils eines weiteren Geflechtendes verbunden sind, zumindest im Bereich nahe der Verbindung der Drahtenden oder zumindest im Bereich nahe der Verbindung der Geflechtenden, denselben Flechtwinkel auf. Das bedeutet, dass Drähte oder aus mehreren Drähten bestehende Geflechtenden in gegenläufiger Richtung unter dem gleichen Winkel im Bereich der jeweiligen Überkreuzung zusammenlaufen. Die sich damit zumindest bereichsweise ergebende symmetrische Struktur des Implantats erleichtert das Crimpen des Implantats, wobei Verzerrungen des Geflechts im Wesentlichen vermieden werden.

Es ist auch möglich, dass zwei, drei oder mehr als drei Drähte und/oder Geflechtenden pro Verbindung denselben Flechtwinkel aufweisen. Die Drähte und/oder Geflechtenden mit demselben Flechtwinkel können gruppiert sein, insbesondere paarweise oder mehrfach, wobei in einer Gruppe mit selbem Flechtwinkel verschiedene, insbesondere entgegengesetzte Windungsrichtungen zusammengefasst sein können. Pro Verbindung können mehrere Gruppen, insbesondere zwei, drei oder mehr Gruppen mit verschiedenen Flechtwinkeln kombiniert sein.

Die Drähte im Bereich der Verbindung der Drahtenden und die ersten Geflechtenden können unterschiedliche Flechtwinkel aufweisen. Die Drähte im Bereich der Verbindung der Drahtenden können also untereinander denselben Winkel, jedoch einen anderen Winkel als die nachgeordneten gebündelten ersten Geflechtenden aufweisen. Dasselbe gilt für die in axial nachgeordneten Umfangsabschnitten vorgesehenen Geflechtenden, die untereinander denselben Flechtwinkel, jedoch einen anderen Flechtwinkel als die axial nachgeordneten Geflechtenden aufweisen können. Bei dieser Ausführungsform sind die Drähte und die aus diesen Drähten gebildeten ersten Geflechtenden in längsaxialer Richtung mit unterschiedlichen Flechtwinkeln angeordnet. Dasselbe gilt für Geflechtenden, die in axial nachgeordneten Umfangsabschnitten vorgesehen sind. Allgemein kann der Flechtwinkel in längsaxialer Richtung unterschiedlich sein, insbesondere zwischen verschiedenen Umfangsabschnitten.

Durch die in längsaxialer Richtung unterschiedlichen Flechtwinkel können die Eigenschaften des Implantants bereichsweise variiert werden.

Bei einer besonders bevorzugten Ausführungsform weisen zwei miteinander verbundene Geflechtenden wenigstens ein Geflechtende auf, das angrenzend an den Verbindungsbereich zwischen den beiden verbundenen Geflechtenden gekrümmt ist. Dadurch wird erreicht, dass ein Geflechtende selbst in Biegung geht und die Verbindungsstelle zwischen den beiden Geflechtenden bezogen auf die beiden Geflechtenden asymmetrisch angeordnet ist. Durch die Krümmung des Geflechtendes wird die Rückstellkraft bzw. die nach außen wirkende Radialkraft im Bereich des Implantatendes erhöht.

Dabei können die Drähte des gekrümmten Geflechtendes verdrillt oder gewickelt sein. Dies hat den Vorteil, dass sich kein Draht komplett auf dem Außenrand der Biegung befindet, so dass kein Draht übermäßig gebogen wird, sondern sich die Biegung gleichmäßig auf alle Drähte verteilt. Insbesondere bei kleinen Drähten hat dies den Vorteil, dass diese der Biegung gut folgen, wodurch ein gutes Verhältnis zwischen Biegeradius und Drahtquerschnitt erreicht wird. Dies führt zu einer hohen Flexibilität und guten Crimpbarkeit des Implantats. Außerdem wird die Rückstellkraft durch die Verdrillung bzw. Wicklung mehrerer Drähte erhöht, die insgesamt zur Gesamtkraft beitragen.

Bei einer anderen Ausführungsform weisen die Drähte, deren Drahtenden zur Bildung jeweils eines ersten Geflechtendes verbunden sind, dieselbe Richtung auf. Das bedeutet, dass die Drähte eines Geflechtendes im Bereich der Verbindung, insbesondere vor der Zusammenführung, bzw. Verbindung zum Geflechtende alle in der gleichen Richtung verlaufen. Diese Ausführungsform ist besonders geeignet in Kombination mit der erfindungsgemäßen Anordnung des zweiten Geflechtendes in Umfangsrichtung der Wandung. Dadurch wird erreicht, dass die Drähte tangential zueinander zusammenlaufen und zu dem ersten Geflechtende verbunden werden, wodurch Verformungen bzw. Beschädigungen der Drähte beim Komprimieren in kleine Zufuhrsysteme vermieden werden.

Dabei können wenigstens zwei erste Geflechtenden, die aus jeweils vor der Verbindung zum Geflechtende gleichgerichteten Drähten gebildet sind, in entgegen gesetzten Richtungen angeordnet und zur Bildung des zweiten Geflechtendes verbunden sein. Dadurch wird ein geschlossenes Implantatende erreicht, das sich im Wesentlichen in Umfangsrichtung ohne axial hervorstehende Komponenten erstreckt.

In einem Geflechtende können 3, insbesondere 4, insbesondere 6, insbesondere 8, insbesondere 12, insbesondere mehr als 12 Drähte vorgesehen sein. Es ist möglich, dass 2, insbesondere 3, insbesondere 4, insbesondere 5, insbesondere 6, insbesondere 7, insbesondere 8, insbesondere 12, insbesondere 16, insbesondere mehr als 16 Geflechtenden vorgesehen sind. Der Flechtwinkel im Bereich der Geflechtenden bzw. unmittelbar vor den Geflechtenden kann größer als 10°, insbesondere größer als 20°, insbesondere größer als 30°, insbesondere größer als 40°, insbesondere größer als 50°, insbesondere größer als 60°, insbesondere größer als 70° sein.

Bei einer weiteren Ausführungsform sind wenigstens zwei Geflechtenden durch ein Verbindungselement miteinander verbunden. Das Verbindungselement gibt die Möglichkeit, die Spannung zwischen den Geflechtenden und somit die Rückstellkraft zu beeinflussen. Vorteilhafterweise ist das Verbindungselement elastisch, so dass dieses beim Crimpen des Stents eine elastische Verformung erfährt. Beim Expandieren des Stents führt die Rückstellkraft des Verbindungselements zu einer Ausweitung der Geflechtenden in die ursprüngliche Position.

Vorteilhafterweise ist das Verbindungselement von einem expandierten Zustand in einen komprimierten Zustand überführbar, so dass die Rückstellkraft im Bereich der Geflechtenden einstellbar ist. Dabei beaufschlagt das Verbindungselement vorteilhafterweise im expandierten Zustand die durch das Verbindungselement verbundene Geflechtenden mit einer Kraft derart, dass die Geflechtenden auseinander gedrückt werden. Dadurch wird die Radialkraft des Implantats beim Expandieren erhöht. Es ist auch möglich, dass das Verbindungselement lediglich die Funktion der Verbindung erfüllt, und im gecrimpten Zustand keine Deformation erfährt.

Das Verbindungselement kann ein Verbindungscoil, eine Verbindungshülse, Verbindungsdrähte oder andere ähnliche Bauteile umfassen.

Vorzugsweise umfasst das Verbindungselement einen mittleren Abschnitt mit Verbindungsenden, wobei die Verbindungsenden mit wenigstens zwei Geflechtenden verbunden sind. Dieser Aufbau des Verbindungselements ermöglicht eine Funktionstrennung, wobei der mittlere Abschnitt beispielsweise dazu konzipiert ist, eine hohe Radialkraft zu erzeugen und die Verbindungsenden für eine sichere Verbindung des mittleren Abschnitts mit den Geflechtenden sorgen.

Der mittlere Abschnitt kann einen im Gebrauch bügelförmigen Draht und/oder ein im Gebrauch bügelförmiges Coil umfassen. Die bügelförmige Ausgestaltung liegt zumindest im Gebrauch vor. Dies kann durch ein bügelförmig vorgeformtes Verbindungselement, das in seiner Ruhestellung, also ohne äußere Krafteinwirkung bügelförmig ist, erfolgen. Alternativ kann der Draht oder das Coil, bzw. allgemein das Verbindungselement die gekrümmten Form durch die Verbindung mit den Geflechtenden annehmen. Das jeweilige Element kann im Ruhezustand, also ohne äußere Krafteinwirkung z.B. gestreckt sein. Allgemein kann das Element im Ruhezustand breiter als der Abstand der zu verbindenden Geflechtenden sein, bspw. indem der Abstand zwischen den Bügelenden im unverbundenen Zustand größer ist als der Abstand zwischen den Geflechtenden, so dass die Verbindung gegen die Federkraft des Elements zu bewerkstelligen ist. Das führt dazu, dass das Verbindungselement eine Restkraft auf die Geflechtenden ausübt, wenn der Stent im Ruhezustand ist und eine Verstärkung der axialen Stentenden, bzw. Implantatenden erreicht wird.

Durch die beispielhafte Ausbildung des Verbindungselementes als Mehrfachverbinder, der mehr als zwei Geflechtenden verbindet, wird eine weitere Möglichkeit zur Verfügung gestellt, die Geflechtenden auf effiziente Weise miteinander zu verbinden.

Die Erfindung wird nachfolgend anhand von Ausführungsbeispielen unter Bezug auf die beigefügten schematischen Zeichnungen näher erläutert. In diesen zeigen:
- Fig. 1: einen Ausschnitt des Axialendes eines Stents nach einem erfindungsgemäßen Ausführungsbeispiel mit entgegengerichtet verbundenen Geflechtenden;
- Fig. 2: einen Ausschnitt des Axialendes eines Stents nach einem weiteren erfindungsgemäßen Ausführungsbeispiel mit gleichgerichtet verbundenen Geflechtenden;
- Fig. 3: einen Ausschnitt des Axialendes eines Stents nach einem weiteren erfindungsgemäßen Ausführungsbeispiel, bei dem die Drähte unterschiedliche Flechtwinkel aufweisen;
- Fig. 4: einen Ausschnitt des Axialendes eines Stents nach einem weiteren erfindungsgemäßen Ausführungsbeispiel, bei dem die Flechtwinkel auf Höhe der Verbindung gleich sind;
- Fig. 5: einen Ausschnitt des Axialendes eines Stents nach einem weiteren erfindungsgemäßen Ausführungsbeispiel, bei dem die Flechtwinkel in längsaxialer Richtung unterschiedlich sind;
- Fig. 6a, 6b: ein Beispiel für die Veränderung der Gitterstrukturen beim Crimpen;
- Fig. 7: einen Ausschnitt des Axialendes eines Stents nach einem weiteren erfindungsgemäßen Ausführungsbeispiel, bei dem mehr als zwei Umfangsabschnitte vorgesehen sind, die durch die Verbindung von Geflechtenden gebildet sind;
- Fig. 8: den Stent gemäß Fig. 7, wobei zusätzlich ein Verbindungselement zwischen den axial außen angeordneten Geflechtenden angeordnet ist;
- Fig. 9: einen Ausschnitt des Axialendes eines Stents nach einem von der beanspruchten Erfindung unabhängigen Ausführungsbeispiel, bei dem die Drähte tangential zu Geflechtenden verbunden sind und in der gleichen Richtung verlaufen;
- Fig. 10: den Stent gemäß Fig. 9, wobei die Verbindung zweiter Geflechtenden dargestellt ist;
- Fig. 11: einen größeren Ausschnitt des axialen Endes des Stents gemäß Fig. 10;
- Fig. 12: einen Ausschnitt des Axialendes eines Stents nach einem weiteren von der beanspruchten Erfindung unabhängigen Ausführungsbeispiel, bei dem die tangential zusammenlaufenden Drahtenden durch ein Verbindungselement verbunden sind;
- Fig. 13: den Stent gemäß Fig. 12, wobei die Drahtenden bügelförmig angeordnet sind;
- Fig. 14a, 14b, 14c: einen Ausschnitt des Axialendes eines Stents nach einem weiteren erfindungsgemäßen Ausführungsbeispiel, bei dem das Verbindungselement ein Verbindungscoil ist;
- Fig. 15: einen Ausschnitt zweier Geflechtenden, bei denen die Verbindungsabschnitte entgegengerichtet angeordnet sind;
- Fig. 16: die Verbindungsabschnitte gemäß Fig. 15 mit einem Verbindungselement;
- Fig. 17: zwei Verbindungsabschnitte in parallel überlappender entgegen gerichteter Stellung;
- Fig. 18: zwei Verbindungsabschnitte, die unter einem Winkel zueinander angeordnet sind;
- Fig. 19: zwei Verbindungsabschnitte, die gleichgerichtet angeordnet sind;
- Fig. 20: ein Verbindungselement, das zwischen zwei Geflechtenden angeordnet ist;
- Fig. 21: einen Ausschnitt des Axialendes eines Stents nach einem erfindungsgemäßen Ausführungsbeispiel, bei dem mehrere Verbindungselemente gemäß Fig. 20 in unterschiedlichen Höhenlagen angeordnet sind;
- Fig. 22: eine Variante des Verbindungselements;
- Fig. 23: eine weitere Variante des Verbindungselements;
- Fig. 24: die Ausbildung des Verbindungselements als Mehrfachverbinder;
- Fig. 25: eine Verbindung zweiter Geflechtenden, von denen ein Geflechtende gekrümmt ist;
- Fig. 26: einen Ausschnitt des Axialendes eines Stents nach einem weiteren erfindungsgemäßen Ausführungsbeispiel mit sich gegenseitig stabilisierenden Endschlaufen;
- Fig. 27: einen Ausschnitt des Stents gemäß Fig. 26 zur Verdeutlichung der Schlaufenstruktur;
- Fig. 28: einen Ausschnitt des Axialendes eines Stents nach einem weiteren erfindungsgemäßen Ausführungsbeispiel mit einer in mehreren Ebenen ausgebildeten Schlaufenstruktur;
- Fig. 29: einen Ausschnitt des Axialendes eines Stents nach einem weiteren erfindungsgemäßen Ausführungsbeispiel mit überlappender Schlaufenstruktur;
- Fig. 30a, 30b: schematische Querschnittsansichten eines Stents mit schlaufenstabilisierten Endbereichen;
- Fig. 31a - d: Darstellungen von Stentenden ohne schlaufenstabilisierte Konfiguration und deren Verhalten bei Verformung.

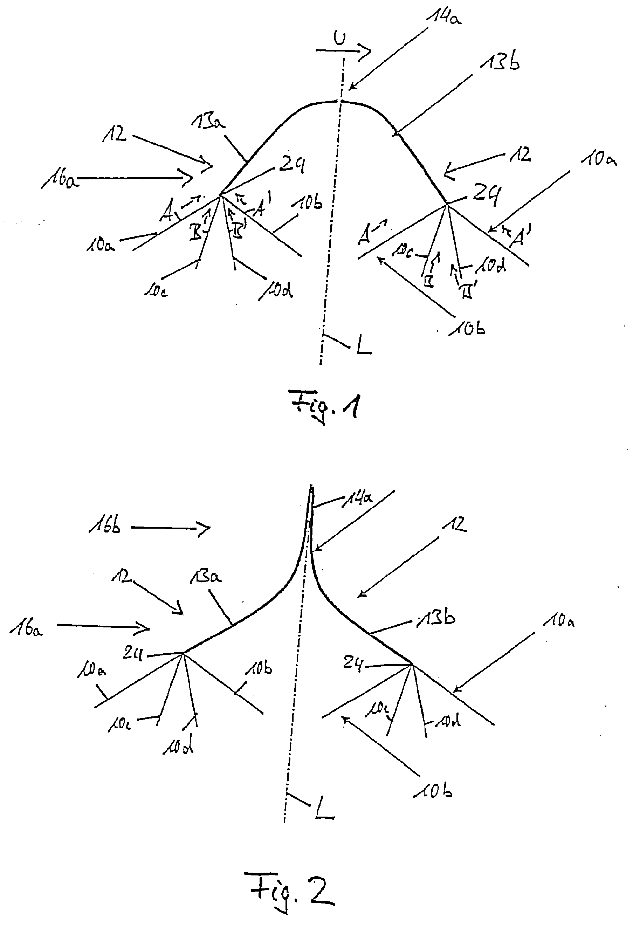

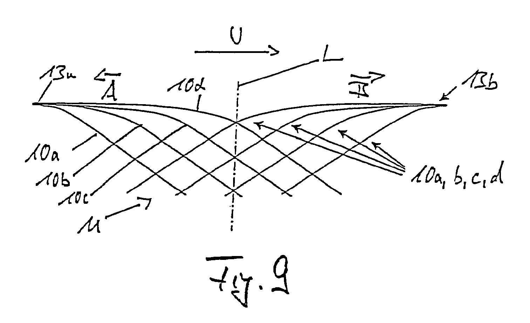

In den Figuren 1, 2 sind zwei verschiedene Beispiele für Implantatenden gezeigt, die beispielsweise bei Stents, insbesondere selbstexpandierbaren Stents, zum Einsatz kommen können. Die Darstellungen gemäß Figuren 1, 2, wie auch die übrigen Darstellungen der Ausführungsbeispiele in den Figuren zeigen der Übersicht halber einen Ausschnitt des axialen Endes eines Stents. Dabei handelt es sich um einen geflochtenen Stent, dessen Wandung 11 (siehe Fig. 9, 10, 11) eine Vielzahl einzelner Drähte bzw. Filamente umfasst, die dieselbe Windungs- oder Spiralrichtung aufweisen und jeweils axial relativ zueinander in Längsrichtung des Stents versetzt sind. Diese Drähte bzw. Filamente kreuzen mit einer entsprechenden Anzahl weiterer Einzeldrähte bzw. Filamente, die in entgegengesetzter Richtung gewunden sind, also eine andere Windungs- oder Spiralrichtung aufweisen und ebenfalls in Axialrichtung des Stents versetzt zueinander angeordnet sind. Diese in an sich bekannter Weise geflochtene Wandung bildet ein Rundgeflecht, das mittels eines geeigneten Zufuhrsystems, beispielsweise eines Einführungskatheters, in einem Gefäß, insbesondere einem Blutgefäß, implantiert werden kann. Dazu ist der Querschnitt des Rundgeflechts in an sich bekannter Weise veränderbar, so dass dieser in das Zuführsystem eingeführt und im Blutgefäß nach Entlassung aus dem Zufuhrsystem expandiert.

Die Erfindung ist nicht auf Stents eingeschränkt, sondern umfasst im Allgemeinen medizinische Implantate, die eine aus mehreren Drähten geflochtene Wandung aufweisen, die sich entlang einer Längsachse L erstreckt und zumindest abschnittsweise um die Längsachse L gekrümmt ist. Dies können beispielsweise Strömungsteiler oder andere rotationssymmetrische Implantate sein. Es ist auch möglich, dass im Ruhezustand flache Implantate auf der Erfindung beruhen, die erst beim Implantieren in die gekrümmte Form gebracht werden.

Die Erfindung ist ferner nicht auf Stents oder Implantate eingeschränkt, deren Geflecht aus Einzeldrähten hergestellt ist. Vielmehr sind auch Implantate umfasst, deren Geflecht aus mehrfachen bzw. gefachten Filamenten bzw. Drähten geflochten sind.

Die Stents gemäß Figuren 1, 2 weisen axiale Enden auf, die durch die Drahtenden 12 der Drähte 10a, 10b, 10c, 10d gebildet sind, wobei die Drahtenden 12 auf dem Umfang des jeweiligen Axialendes des Stents angeordnet sind. Die Darstellungen in diesen und den übrigen beigefügten Figuren zeigen jeweils Ausschnitte der Axialenden von Stents. Die zugehörige geflochtene Wandung 11 ist aus Gründen der Übersichtlichkeit weggelassen und nur in den Figuren 9 - 11 bereichsweise dargestellt. Die in den Figuren dargestellten Verbindungen der verschiedenen Geflechtenden sind auf dem Umfang der Axialenden der Stents verteilt angeordnet. Die Erfindung ist nicht auf die dargestellten einzelnen Verbindungen der Geflechtenden eingeschränkt, sondern umfasst Implantate, insbesondere Stents, bei denen mehrere dieser Verbindungen auf dem Umfang eines Axialendes nebeneinander und/oder in Längsrichtung nachgeordnet vorgesehen sind.

Bei dem Ausführungsbeispiel gemäß Fig. 1 ist gut zu erkennen, dass jeweils wenigstens zwei Drahtenden 12, bei dem Ausführungsbeispiel gemäß Fig. 1 konkret 4 Drahtenden, der Drähte 10a, 10b, 10c, 10d zu wenigstens zwei ersten Geflechtenden 13a, 13b verbunden sind. Die Drähte 10a, 10b, 10c, 10d sind im Geflecht der Wandung 11 teilweise miteinander und/oder mit weiteren Drähten verflochten. Im distalen Bereich vor dem jeweiligen ersten Geflechtende 13a, 13b sind die Drähte 10a, 10b, 10c, 10d voneinander beabstandet angeordnet und proximal zum jeweiligen ersten Geflechtende 13a, 13b hin zusammengeführt. Die Drähte 10a, 10b, 10c, 10d treffen sich im ersten Überkreuzungspunkt 24 und sind zur Bildung des ersten Geflechtendes 13a, 13b verbunden. Zur Verbindung der Drahtenden 12 zum ersten Geflechtende 13a, 13b können die Drahtenden 12 beispielsweise verdrillt und/oder gewickelt sein, auch abschnittsweise verdrillt und gewickelt. Sie können auch parallel zueinander oder in einer beliebigen anderer Konfiguration verlaufen und können miteinander durch Klebe-, Schweiß- oder Löttechnik oder durch ein weiteres Element, wie z.B. eine Hülse zusammengehalten werden. Das erste Geflechtende 13a, 13b umfasst somit ein Bündel oder eine Gruppe von verbundenen Drahtenden 12, die aus den Drähten 10a, 10b, 10c, 10d gebildet sind. Dies ist zeichnerisch durch den etwas dickeren Strich für die ersten Geflechtenden 13a, 13b dargestellt.

Dies gilt für alle Ausführungsbeispiele der Anmeldung.

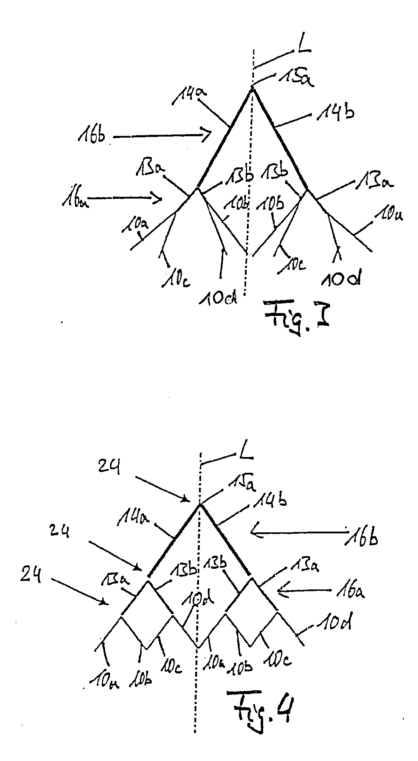

Wie bei dem Ausführungsbeispiel gemäß Fig. 1 ist auch bei dem Ausführungsbeispiel gemäß Fig. 2 vorgesehen, dass die Drähte 10a, 10b, 10c, 10d, deren Drahtenden 12 zur Bildung jeweils eines Drahtendes 13a, 13b verbunden sind, gegenläufige Richtungen A, A', B, B' aufweisen. Unter gegenläufigen Richtungen sind entgegengesetzte Spiral- oder Windungsrichtungen der Drähte zu verstehen. Konkret weisen die unmittelbar nebeneinander angeordneten Drähte 10a, 10c Spiralrichtungen A, B auf, die gegenläufig zu den Spiralrichtungen A', B' beiden anderen unmittelbar nebeneinander angeordneten Drähten 10b, 10d sind. Die Drähte 10a, 10b, 10c, 10d sind paarweise angeordnet, wobei jeweils ein Drahtpaar gegenläufige Windungsrichtungen aufweist. Mehrere Drahtpaare pro Geflechtende sind möglich. Die Ausführungsbeispiele gemäß den Figuren 3 bis 8 sind diesbezüglich entsprechend aufgebaut.

Die Erfindung ist nicht auf 4 Drahtenden 12 bzw. 4 Drähte 10a, 10b, 10c, 10d pro Geflechtende 13a, 13b eingeschränkt, sondern kann 3, 4, 6, 8, 12 und mehr als 12 Drähte 10a, 10b, 10c, 10d pro Geflechtende 13a, 13b, 14a, 14b, 15a, 15b umfassen. Die Erfindung ist auch nicht auf die Verbindung von zwei Geflechtenden eingeschränkt, sondern kann mehr als zwei Geflechtend-Paare umfassen. Insbesondere kann die Erfindung 2, 4, 5, 7, 8, 12 und mehr als 12 Geflechtenden 13a, 13b, 14a, 14b, 15a, 15b umfassen.

Dies gilt für alle Ausführungsbeispiele, die in dieser Anmeldung offenbart sind.

Um die Verbindung der ersten Geflechtenden zu wenigstens einem weiteren Geflechtende zu ermöglichen, sind mindestens zwei erste Geflechtenden 13a, 13b erforderlich.

Wie in Fig. 1 zu erkennen, bilden die ersten Geflechtenden 13a, 13b einen um die Längsachse L erstreckten ersten Umfangsabschnitt 16a der Wandung 11. Der erste Umfangsabschnitt 16a begrenzt eine rotationssymmetrische, insbesondere zylindrische Hüllkurve, die im Bereich des axialen Endes des Stents dessen Lumen bestimmt. Auf dieser imaginären Hüllkurve bzw. auf dem ersten Umfangsabschnitt 16a können mehrere erste Geflechtenden 13a, 13b, die jeweils, insbesondere paarweise miteinander verbunden sind, angeordnet sein.

Der erste Umfangsabschnitt 16a ist stentseitig durch den Überkreuzungspunkt 24 begrenzt. Axial außen ist der erste Umfangsabschnitt 16a durch die Außenkontur des Stents begrenzt und bildet den Außenrand des Stents.

Wie in Fig. 1 dargestellt, sind die beiden ersten Geflechtenden 13a, 13b symmetrisch zur Längsachse des Stents angeordnet. Eine asymmetrische Anordnung ist ebenfalls möglich. Die Geflechtenden 13a, 13b gemäß Fig. 1 sind konvex aufeinander zugebogen und miteinander verbunden. Durch die Verbindung der beiden ersten Geflechtenden 13a, 13b wird ein zweites Geflechtende gebildet, das mit dem Bezugszeichen 14a bezeichnet ist. Bei dem Ausführungsbeispiel gemäß Fig. 1 bildet das zweite Geflechtende 14a zugleich den Abschluss des Stents in axialer Richtung. Dazu ist das zweite Geflechtende 14a in Umfangsrichtung U der Wandung 11 angeordnet und umfasst den Verbindungsbereich der beiden ersten Geflechtenden 13a, 13b. Die beiden ersten Geflechtenden 13a, 13b verlaufen im Randbereich des axialen Stentendes tangential zur Umfangsrichtung U. Die Geflechtenden 13a, 13b sind entgegengerichtet angeordnet.

Im Unterschied zu dem Ausführungsbeispiel gemäß Fig. 1 sind bei dem Ausführungsbeispiel gemäß Fig. 2 die Geflechtenden 13a, 13b im Bereich der Verbindung gleichgerichtet angeordnet. Dazu sind die beiden ersten Geflechtenden 13a, 13b konkav angeordnet und bilden einen ersten Umfangsabschnitt 16a sowie einen dem ersten Umfangsabschnitt 16a in längsaxialer Richtung nachgeordneten zweiten Umfangsabschnitt 16b. Der erste Umfangsabschnitt 16 ist, wie beim Ausführungsbeispiel mit Fig. 1, stentseitig durch den Überkreuzungspunkt 24 begrenzt, an dem die Drähte 10a, 10b, 10c, 10d zusammenlaufen und zum ersten Geflechtende 13, 13b verbunden sind. Der zweite Umfangsabschnitt 16b befindet sich im Bereich der Verbindung zwischen den beiden ersten Geflechtenden 13a, 13b. Der zweite Umfangsabschnitt 16b wird somit durch das die beiden ersten Geflechtenden 13a, 13b gebildete zweite Geflechtende 14a gebildet. Das zweite Geflechtende 14a ist somit den beiden ersten Geflechtenden 13a, 13b ebenfalls in längsaxialer Richtung nachgeordnet. Der Übergang zwischen dem ersten Umfangsabschnitt 16a und dem zweiten nachgeordneten Umfangsabschnitt 16b ist bei dem Ausführungsbeispiel gemäß Fig. 2 kontinuierlich. Die beiden Umfangsabschnitte 16a, 16b unterscheiden sich im Wesentlichen dadurch, dass der erste Umfangsabschnitt 16a durch Geflechtenden 13a, 13b gebildet ist, deren Erstreckung eine Längskomponente und eine Umfangskomponente aufweist. Das zweite Geflechtende 14a, das den zweiten Umfangsabschnitt 16b bildet, weist dagegen im Wesentlichen nur eine Längskomponente in Axialrichtung des Stents auf.

Weitere Ausführungsbeispiele, bei denen die Drähte 10a, 10b, 10c, 10d erste Geflechtenden bilden, die wiederum miteinander zu zweiten Geflechtenden 14a, 14b verbunden sind, sind in den Figuren 3 bis 6 dargestellt. Diesen Ausführungsbeispielen ist gemeinsam, dass jeweils zwei Drähte 10a, 10c bzw. 10d, 10b jeweils zu einem ersten Geflechtende 13a, 13b verbunden sind. Die Verbindung erfolgt, wie bei den Ausführungsbeispielen gemäß Figuren 1, 2 im Bereich eines ersten Überkreuzungspunktes 24. Die beiden ersten Geflechtenden 13a, 13b sind also jeweils aus zwei Drahtenden gebildet und dementsprechend in den Fig. 3 bis 6 durch einen dickeren Strich gekennzeichnet, als die einzelnen Drähte 10a, 10b, 10c, 10d. Die beiden ersten Geflechtenden 13a, 13b, konkret sind dies in den Fig. 3 bis 6b vier erste Geflechtenden 13a, 13b, sind paarweise zusammengeführt und zu einem zweiten Geflechtenden 14a, 14b verbunden. Die Zusammenführung erfolgt im zweiten Überkreuzungspunkt 24, der dem ersten Überkreuzungspunkt 24 in längsaxialer Richtung nachgeordnet ist. Konkret sind zwei zweite Geflechtenden 14a, 14b vorgesehen. Diese wiederum sind im dritten Überkreuzungspunkt 24, der dem zweiten Überkreuzungspunkt 24 in längsaxialer Richtung nachgeordnet ist, verbunden und bilden das dritte Geflechtende 15a. Das dritte Geflechtende 15a begrenzt die Außenkonturen des axialen Stentendes.

Zur begrifflichen Unterscheidung weisen die ersten Geflechtenden 13a, 13b und die zweiten Geflechtenden 14a, 14b jeweils eine Längserstreckung auf, die eine Axialund eine Umfangskomponente aufweist. Das abschließende dritte Geflechtende 15a bildet die Verbindung der beiden zweiten Geflechtenden 14a, 14b und somit den Rand des Stents. Das dritte Geflechts-Ende 15a weist in diesem Sinne keine Längserstreckung auf, wie die ersten und zweiten Geflechtenden 13a, 13b, 14a, 14b, sondern bezeichnet die Verbindungsstelle zwischen den beiden zweiten Geflechtenden 14a, 14b. Dies gilt generell für abschließende Geflechtenden, die das Axialende des Implantats bzw. Stents bilden.

In den Figuren 3, 4 ist der Aufbau des axialen Stentendes mit zwei in längsaxialer Richtung nachgeordneten Umfangsabschnitten 16a, 16b zu erkennen, wobei der erste Umfangsabschnitt 16a durch die beiden ersten Geflechtenden 13a, 13b und der zweite Umfangsabschnitt 16b durch die beiden zweiten Geflechtenden 14a, 14b gebildet ist.

Bei dem Ausführungsbeispiel gemäß Fig. 3 weisen die Drahtpaare 10a, 10c und 10b, 10d, die die beiden ersten Geflechtenden 13a, 13b bilden, unterschiedliche Flechtwinkel auf, woraus sich ein lokal asymmetrisches Gebilde ergibt. Bei dem Ausführungsbeispiel gemäß Fig. 4 weisen die Drähte 10a, 10b, 10c, 10d, deren Drahtenden 12 zur Bildung jeweils des ersten Geflechtendes 13a, 13b verbunden sind, denselben Flechtwinkel auf. Dasselbe gilt für die ersten Geflechtenden 13a, 13b, die - ebenso wie die zweiten Geflechtenden 14a, 14b - jeweils dieselben Flechtwinkel aufweisen. Insgesamt sind die Drähte 10a, 10b, 10c, 10d sowie die ersten und zweiten Geflechtenden 13a, 13b, 14a, 14b alle mit demselben Flechtwinkel ausgerichtet, wodurch sich die in Fig. 4 dargestellte rautenförmige Struktur ergibt.

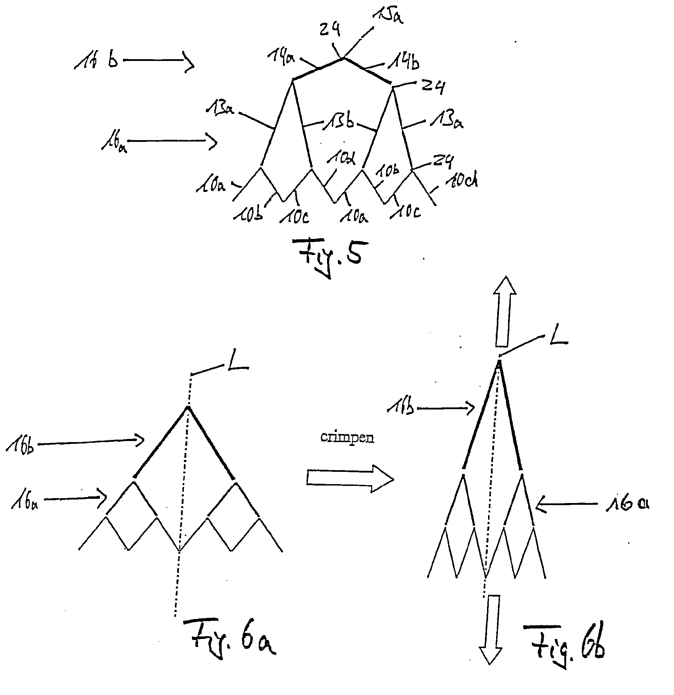

Im Gegensatz zur asymmetrischen Struktur gemäß Fig. 3 hat die symmetrische Struktur gemäß Fig. 4 den Vorteil, dass das Crimpen auf kleine Durchmesser vereinfacht wird. Bei dem asymmetrischen Geflecht neigen die Drähte mit dem größeren Flechtwinkel dazu, sich beim Crimpen mehr als die Drähte mit flachem Winkel in ihrer Spiralrichtung zu verlängern. Die Spirale verlängert sich. Es kommt daher beim Crimpen zur Dehnung der Drähte mit kleinem Winkel und zur Stauchung der Drähte mit großem Winkel. Im Gegensatz dazu werden die Drähte und Geflechtenden gemäß Fig. 4 beim Crimpen mit derselben Spiralverlängerung beaufschlagt, wodurch eine Verzerrung vermieden wird. Dies ist besonders gut in den Fig. 6a, 6b zu erkennen, wobei in Fig. 6a der Zustand vor dem Crimpen und in Fig. 6b der Zustand nach dem Crimpen dargestellt ist. Durch die in Stent-Längsrichtung verlaufenden Pfeile ist die Längenänderung des Stents beim Crimpen verdeutlicht.

Bei dem Ausführungsbeispiel gemäß Fig. 5 ist zu erkennen, dass die auf demselben Umfangsabschnitt angeordneten Elemente denselben Flechtwinkel aufweisen. Die Elemente verschiedener Umfangsabschnitte weisen unterschiedliche Flechtwinkel auf. In Längsrichtung des Stents variiert daher der Flechtwinkel. Konkret weisen die Drähte 10a, 10b, 10c, 10d einen anderen Flechtwinkel auf, als die ersten Geflechtenden 13a, 13b, die wiederum einen anderen Flechtwinkel aufweisen, als die zweiten Geflechtenden 14a, 14b. Beispielsweise ist der Flechtwinkel der zweiten Geflechtwinkel 14a, 14b größer als der Flechtwinkel der ersten Geflechtenden 13a, 13b, der wiederum kleiner ist als der Flechtwinkel der Drähte 10a, 10b, 10c, 10d. Andere Variationen des Flechtwinkels sind möglich.

Vorteilhaft ist bei dem Ausführungsbeispiel gemäß Fig. 5, dass, wie auch beim Ausführungsbeispiel gemäß Fig. 4, die Drähte 10a, 10b, 10c, 10d bzw. allgemein die Elemente, die in einem Überkreuzungspunkt 24 zusammenlaufen, untereinander denselben Flechtwinkel aufweisen.

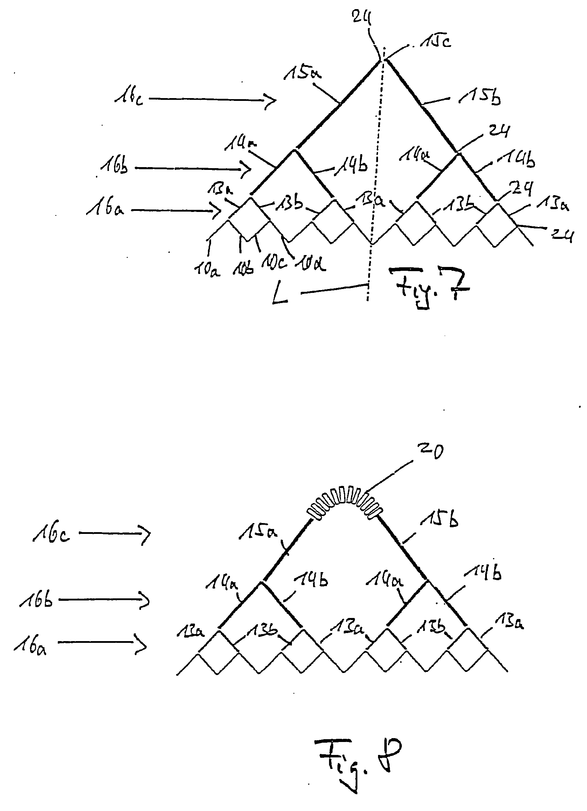

Das Ausführungsbeispiel gemäß Fig. 7 zeigt, wie das erfindungsgemäße Prinzip der Verbindung der Geflechtenden in Längsrichtung des Stents fortgesetzt wird. Der Stent gemäß Fig. 7 weist drei Umfangsabschnitte 16a, 16b, 16c auf, die jeweils durch miteinander verbundenen Geflechtenden gebildet sind. Dabei sind jeweils zwei Drähte 10a, 10b, 10c, 10d zu einem ersten Geflechtende 13a, 13b verbunden, insbesondere verdrillt. Insgesamt umfasst die in Fig. 7 dargestellte Konfiguration 16 Drähte 10a, 10b, 10c, 10d, die zu acht ersten Geflechtenden 13a, 13b zusammengefasst bzw. gebündelt sind. Eine andere Anzahl ist möglich. Die ersten Geflechtenden 13a, 13b sind jeweils zu zweiten Geflechtenden 14a, 14b verbunden, die aus jeweils vier Drähten bzw. Drahtenden gebildet, insbesondere geflochten sind. Insgesamt umfasst der zweite Umfangsabschnitt 16b vier zweite Geflechtenden 14a, 14b. Jeweils zwei zweite Geflechtenden 14a, 14b sind zu einem dritten Geflechtende 15a, 15b verbunden, die den dritten Umfangsabschnitt 16c bilden. Die dritten Geflechtenden 15a, 15b umfassen jeweils acht Drähte und sind beispielsweise verdrillt. Die dritten Geflechtenden 15a, 15b sind zu einem vierten Abschluss-Geflechtende 15c verbunden, das den Rand, bzw das Axialende des Stents begrenzt.

Das Abschluss-Geflechtende ist aus den Verbindungsabschnitten 17a, 17b der vorgeordneten Geflechtenden gebildet, die winkelförmig oder tangential auf dem Umfang zueinander fluchtend angeordnet sein können. Dies gilt für alle Abschluss-Geflechtenden in dieser Anmeldung.

Andere Verbindungsarten der Geflechtenden sind möglich, wobei das Flechten der Geflechtenden eine Verbindung mit erhöhter Stabilität ermöglicht. Das Verdrillen hat den Vorteil, dass die Drähte näher aneinander gebracht werden, so dass der Platzbedarf reduziert wird. Es ist auch möglich, dass zwischen zwei Überkreuzungen 24 sowohl verdrillte als auch geflochtene Bereiche vorgesehen sind. Zum Beispiel kann der an die Überkreuzung 24 angrenzende Bereich der Drähte geflochten und der im Mittelbereich des Segments zwischen den beiden Überkreuzungen 24 verdrillt sein. Sie können auch parallel verlaufen und durch andere Verbindungstechniken, bspw. stoffschlüssig verbunden werden. Diese Verbindungsarten werden für alle in der Anmeldung beschriebenen Ausführungsbeispiele offenbart.

Das Ausführungsbeispiel gemäß Fig. 8 ist wie das Ausführungsbeispiel gemäß Fig. 7 aufgebaut, wobei zusätzlich die beiden dritten Geflechtenden 15a, 15b durch ein Verbindungselement 20 verbunden sind. Das Verbindungselement 20 kann, wie in Fig. 8 dargestellt, ein Coil sein. Es bietet sich an, die Drähte der dritten Geflechtenden 15a, 15b im Verbindungsbereich, d.h. im Bereich des vierten Geflechtendes 15c zu verdrillen, so dass das vierte Geflechtende 15c einen relativ kleinen Querschnitt aufweist, auf dem das Coil oder ein anderes Verbindungselement 20 angebracht werden kann.

Bei den Ausführungsbeispielen gemäß den Fig. 9 bis 11 ist eine andere Variante dargestellt, wie die Drähte 10a, 10b, 10c, 10d zu Geflechtenden 13a, 13b verbunden werden bzw. wie die daraus resultierenden Geflechtenden 13a, 13b zu weiteren Geflechtenden 14a verbunden sind.

Bei dem Ausführungsbeispiel gemäß Fig. 9 sind nur Drähte zu einem ersten Geflechtende 13a, 13b gebündelt, die dieselbe Spiralrichtung A, B aufweisen. Dies hat den Vorteil, dass die jeweils gebündelten Drähte tangential in Umfangsrichtung zusammenlaufen, wodurch abrupte Winkeländerungen und Verformungen der Drähte vermieden werden. Das Ausführungsbeispiel gemäß Fig. 9 wird sowohl unabhängig von dem Prinzip, die Geflechtenden zu verbinden, als auch zusammen mit diesem Prinzip offenbart.

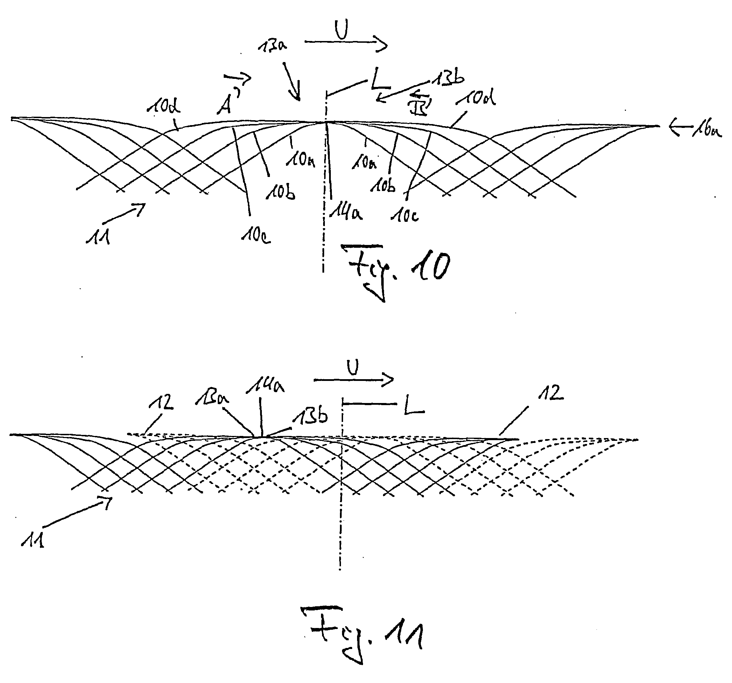

Die Verbindung der durch tangential zusammenlaufende Drähte 10a, 10b, 10c, 10d gebildeten ersten Geflechtenden 13a, 13b ist in Fig. 10 dargestellt. Dort ist zu erkennen, dass jeweils Gruppen von Drähten miteinander verbunden sind, die innerhalb der Gruppe dieselbe Spiralrichtung A', B' aufweisen. Die Gruppen selbst weisen eine entgegen gerichtete Spiralrichtung A', B' auf, so dass die beiden ersten Geflechtenden 13a, 13b, die miteinander verbunden sind, entgegengerichtet angeordnet sind. Die so verbundene Geflechtenden 13a, 13b sind zumindest teilweise oder vorwiegend in Umfangsrichtung U' ausgerichtet und bilden den ersten (und einzigen) Umfangsabschnitt 16a des axialen Endes des Stents.

Die Fortführung des Prinzips gemäß Fig. 10 in Umfangsrichtung U des Stents ist in Fig. 11 dargestellt. Dort ist zu erkennen, dass die Drahtenden 12, die in Umfangsrichtung U angeordnet sind, sich mit Drahtenden 12 kreuzen, die aus der entgegengesetzten Richtung kommen. Die miteinander verbundenen Geflechtenden 13a, 13b, die aus unterschiedlichen Richtungen kommen, sind dabei so miteinander verbunden, dass die jeweiligen Spitzen der Enden aufeinander gerichtet sind. Daraus resultiert eine Schlaufenkonfiguration, die dem Geflecht-Gesamtende eine erhöhte Radialkraft verleiht. Beim Crimpen erfolgt eine Verkleinerung des Radius bzw. Winkels, mit dem die beiden Enden zusammengeführt sind. Dies führt zu einer Verformung der Schlaufe, wodurch elastische Energie gespeichert wird, die beim Entlassen des Geflechts aus dem Zufuhrsystem freigesetzt wird. Dies hat den Vorteil, dass die Gefäßwand geschont wird. Dies hat außerdem den Vorteil der größeren Kraft im Endenbereich des Stents und der daher besseren Anpassung an die Gefäßwand.

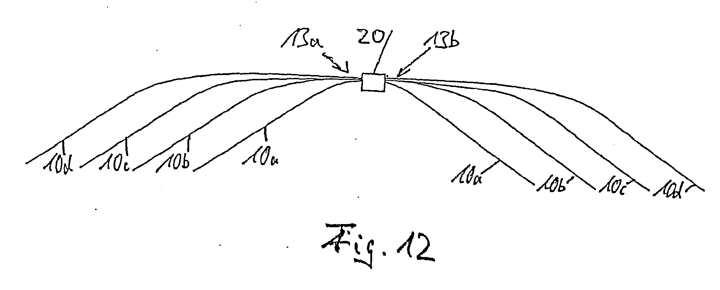

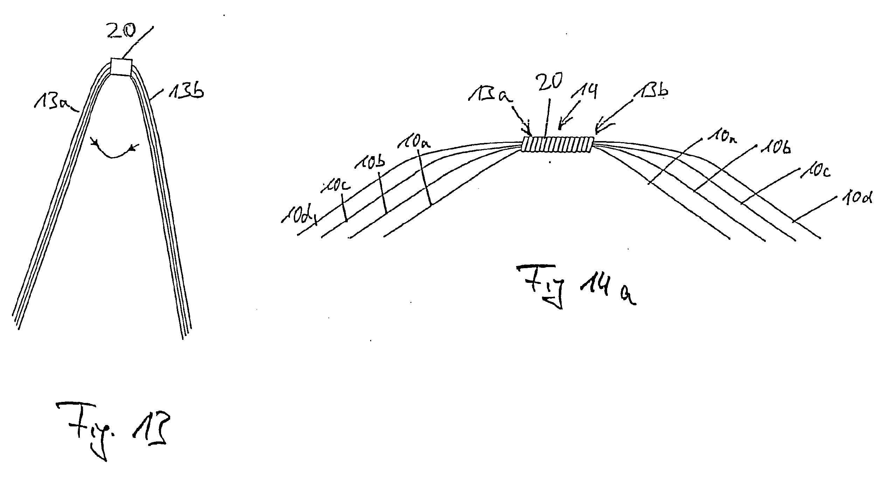

Wie in den Fig. 12, 13 dargestellt, kann die Verbindung zwischen den Geflechtenden 13a, 13b kraft-, form- oder reibschlüssig erfolgen, wobei vorzugsweise ein Verbindungselement 20 eingesetzt wird, das elastisch ist und beim Crimpen des Stents eine elastische Verformung erfährt. Beim Expandieren des Stents führt die Rückstellkraft des Verbindungselements 20 zu einer Ausweitung der Enden in die ursprüngliche Position. Das elastische Element kann beispielsweise als Coil oder als Hülse ausgeführt sein. Das Coil kann bügelartig konfiguriert sein und sich in einem großen Winkelbereich ohne plastische Verformung verbiegen lassen. Andere elastische Verbindungselemente sind ebenfalls möglich, wie beispielsweise Hülsen oder Drähte, aus Kunststoff oder Metall, die sich elastisch verformen lassen und beim Expandieren die gespeicherte Energie in Form von Radialkraft freigeben. Die Elemente können geklebt, geschweißt, gelötet oder kraft-, form- oder reibschlüssig verbunden sein. Beispielsweise können die Verbindungselemente 20 Kunststoffhülsen oder Metalldrähte umfassen. Die Verbindungselemente können aus einem röntgensichtbaren Material hergestellt sein. Sie können auch aus Federstahl oder Nitinol hergestellt sein. Das Verbindungselement kann aus einem Material so geschnitten, gelasert oder geätzt werden, dass das resultierende Profil mit den Geflechtenden verbunden werden kann und evtl. biegbar ist. Das Verbindungselement kann mindestens teilweise ein Hypotube, also ein spiral geschnittenes Rohr sein. Das Verbindungselement kann auch ein Seil umfassen, wobei im Gegensatz zu Drähten keine Verformung beim Crimpen auftritt. Das Verbindungselement kann generell aus Kunststoff, z.B. Polyamid, PTFE, PE hergestellt sein.

Zur Erzeugung der Radialkraft kann das Verbindungselement auch als Formteil ausgebildet sein, das im Ruhezustand des Stents auf die Geflechtenden eine Kraft ausübt. Es ist auch nicht ausgeschlossen, dass die Verbindungselemente eine plastische Verformung erfahren zusätzlich zur elastischen Verformung.

Die vorstehend genannten Verbindungen sind für alle Ausführungsbeispiele in dieser Anmeldung offenbart.

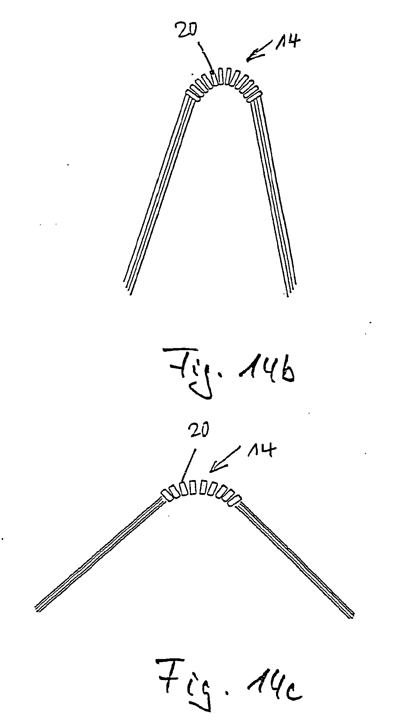

Die Ausbildung des Verbindungselementes als Coil ist in den Fig. 14a, 14b, 14c dargestellt, wobei in Fig. 14a die Verbindung durch ein Coil im expandierten Zustand und in Fig. 14b die Verbindung durch ein Coil im komprimierten Zustand offenbart ist. Das Coil kann bereits im expandierten Zustand eine Bügelform aufweisen, wodurch die Gefäßwand im expandierten Zustand optimal geschont wird (Fig. 14c).

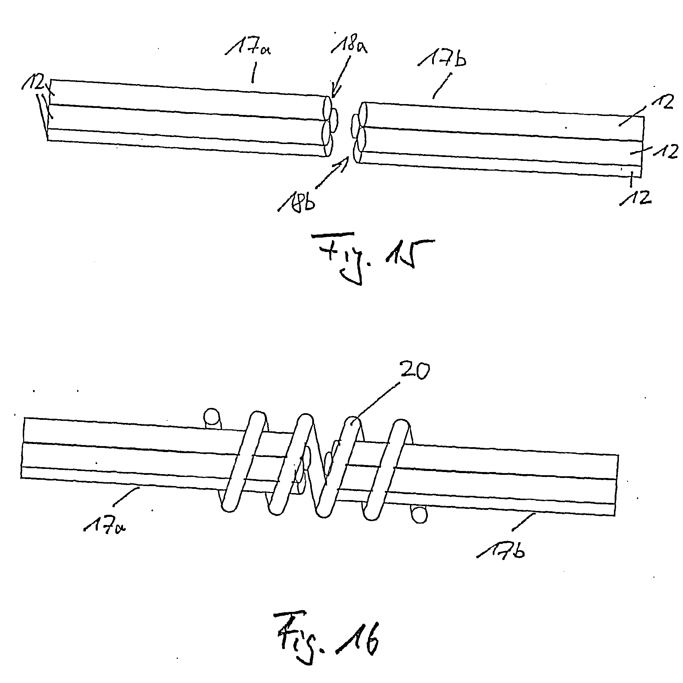

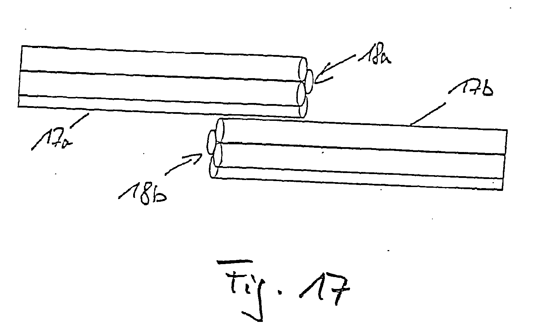

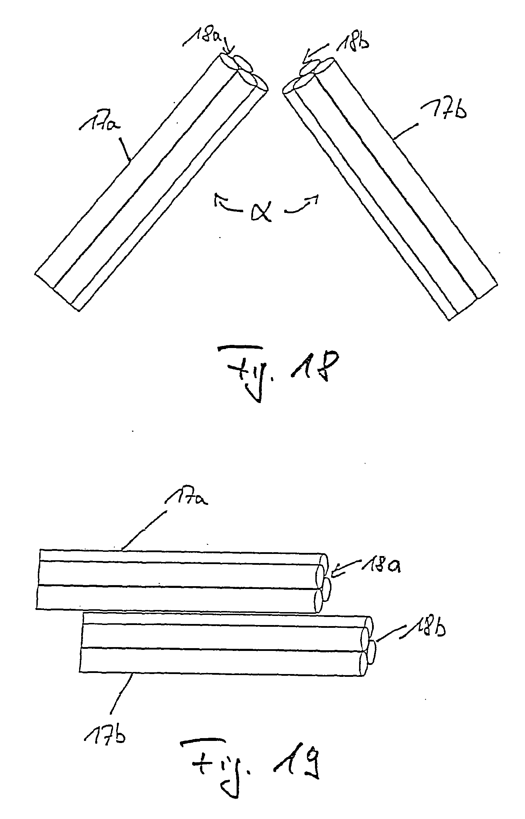

In den Fig. 15 bis 19 sind verschiedene Beispiele dargestellt, wie die Anordnung der miteinander verbundenen Geflechtenden ausgestaltet ist, wobei der Abschnitt, in dem die Geflechtenden zur Verbindung zusammenwirken, als Verbindungsabschnitt 17a, 17b bezeichnet ist. Die Anordnungen gemäß den Fig. 15 bis 18 betreffen die Ausführungsbeispiele, bei denen die Geflechtenden 14a, 14b, 15a, 15b entgegengerichtet angeordnet sind, d.h. die Geflechtenden oder zumindest die Verbindungsabschnitte 17a, 17b kommen aus entgegengesetzten Richtungen, wie bspw. in Fig. 1 dargestellt. Bei dem Ausführungsbeispiel gemäß Fig. 15 sind die Verbindungsabschnitte 17a, 17b mit ihren Stirnflächen 18a, 18b in Gegenüberstellung angeordnet. Die Verbindungsabschnitte 17a, 17b fluchten. Wie in Fig. 16 dargestellt, kann die Verbindung beispielsweise durch ein Verbindungselement 20 erfolgen, das als Coil ausgebildet ist, wobei andere Verbindungselemente oder Verbindungsarten, wie beispielsweise stoffschlüssige Verbindungen, möglich sind. Bei dem Ausführungsbeispiel gemäß Fig. 17 sind die Verbindungsabschnitte 17a, 17b parallel überlappend angeordnet. Bei dem Ausführungsbeispiel gemäß Fig. 18 sind die Verbindungsabschnitte 17a, 17b unter einem Winkel α miteinander verbunden.

Das Ausführungsbeispiel gemäß Fig. 19 ist besonders geeignet in Zusammenhang mit dem Ausführungsbeispiel gemäß Fig. 2, ohne darauf eingeschränkt zu sein, wobei die Verbindungsabschnitte 17a, 17b gleichgerichtet angeordnet und miteinander verbunden sind. Das bedeutet, dass die Verbindungsabschnitte 17a, 17b aus der gleichen Richtung kommen. Das Ausführungsbeispiel gemäß Fig. 19 wird auch in Kombination mit den Ausführungsbeispielen gemäß den Figuren 9 bis 14 offenbart. Die Verbindungsabschnitte 17a, 17b, bzw. die Geflechtenden können somit tangential in Umfangsrichtung angeordnet sein.

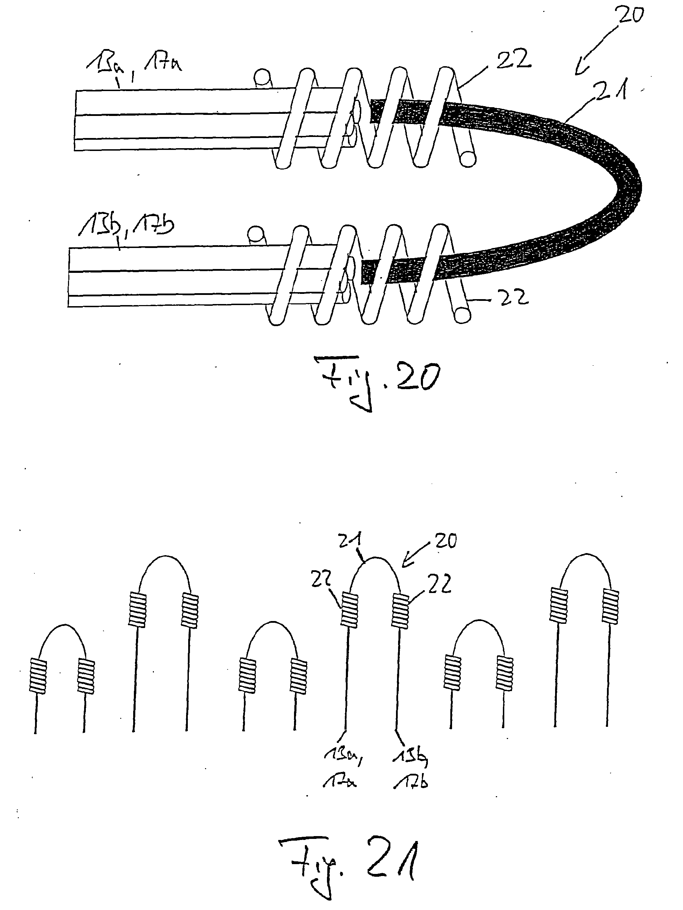

Anhand der Figuren 21 bis 25 werden verschiedene Verbindungsmöglichkeiten beschrieben. Nicht dargestellt sind die Möglichkeiten, die Geflechtenden 13a, 13b, 14a, 14b, 15a, 15b kraft-, form-, stoffschlüssig zu verbinden, die im Zusammenhang mit allen Ausführungsbeispielen offenbart werden. Beispielsweise können die Geflechtenden 13a, 13b, 14a, 14b, 15a, 15b verschweißt, verklebt oder anderweitig verbunden sein.

Bei dem Ausführungsbeispiel gemäß Fig. 20 sind die Verbindungsabschnitte 17a, 17b, also die Endbereiche der Geflechtenden 13a, 13b, 14a, 14b, 15a, 15b, die miteinander verbunden sind, gleichgerichtet. Diese Bereiche, bzw. Abschnitte weisen, bzw. sind in die gleiche Richtung erstreckt. Der übrige Bereich der Geflechtenden 13a, 13b, 14a, 14b, 15a, 15b kann entgegengerichtet sein. Ein Beispiel hierfür ist in Fig. 2 dargestellt. Das Ausführungsbeispiel gemäß Fig. 20 ist auch für die Ausführungsbeispiele gemäß den Figuren 3 bis 8 geeignet und zwar zur Verbindung der in längsaxialer Richtung äußeren, bzw. abschließenden Geflechtenden.

Das in Fig. 20 dargestellte Verbindungselement 20 weist einen mittleren Abschnitt 21 mit Verbindungsenden 22 auf, wobei die Verbindungsenden 22 mit wenigstens zwei Geflechtenden 13a, 13b, 14a, 14b, 15a, 15b verbunden sind bzw. mit den zugehörigen Verbindungsabschnitten 17a, 17b. Das Verbindungselement gemäß Fig. 20 kann mit allen Ausführungsbeispielen dieser Anmeldung kombiniert werden. Das gilt auch für die anderen Verbindungselemente, gemäß den Figuren 21 bis 25. Bei dem Ausführungsbeispiel gemäß Fig. 20 ist der mittlere Abschnitt 21 als bügelförmiger Draht ausgebildet, wobei die Verbindungsenden 22 Coils umfassen, die im verbundenen Zustand sowohl den mittleren Abschnitt 21, d.h. den Draht, als auch die Verbindungsabschnitte 17a, 17b der Geflechtenden 13a, 13b umgreifen und halten. Aus Gründen der Übersichtlichkeit sind die beiden Coils gemäß Fig. 20 mit konstantem Durchmesser dargestellt. Im Haltezustand sind die Coils fest mit dem Draht verbunden. An Stelle der Coils können auch die Drahtenden des mittleren Abschnittes 21 mit den Verbindungsabschnitten 17a, 17b verschweißt, verklebt oder anderweitig stoffschlüssig oder form- oder reibschlüssig verbunden sein. An Stelle der Coils können auch Hülsen oder andere Verbindungsmittel verwendet werden, die mit dem Draht und den Verbindungsabschnitten 17a, 17b verklebt oder verbunden sind. Der mittlere Abschnitt 21 kann mehrere Drähte umfassen, die miteinander verdrillt sind. Aus Gründen der Einfachheit sind die Drähte des Geflechtendes parallel dargestellt. Vorzugsweise sind die Drähte miteinander verbunden, insbesondere verdrillt oder geflochten. Dies gilt für alle Ausführungsbeispiele in dieser Anmeldung.

Bei dem Ausführungsbeispiel gemäß Fig. 21 ist die Anordnung der Verbindungselemente 20 gemäß Fig. 20 anhand eines Stents mit 12 Enden, z.B. aus jeweils vier Drähten, dargestellt. Diese Anordnung ist auf alle anderen Verbindungselemente anwendbar. Die Draht-Elemente sind mittels bogenförmiger elastischer Verbindungselemente 20 verbunden, wobei die Verbindungselemente 20 versetzt über den Umfang verteilt angeordnet sind. Dadurch wird erreicht, dass beim Crimpen im Zufuhrsystem der gesamte Platzbedarf auf dem Umfang klein ist.

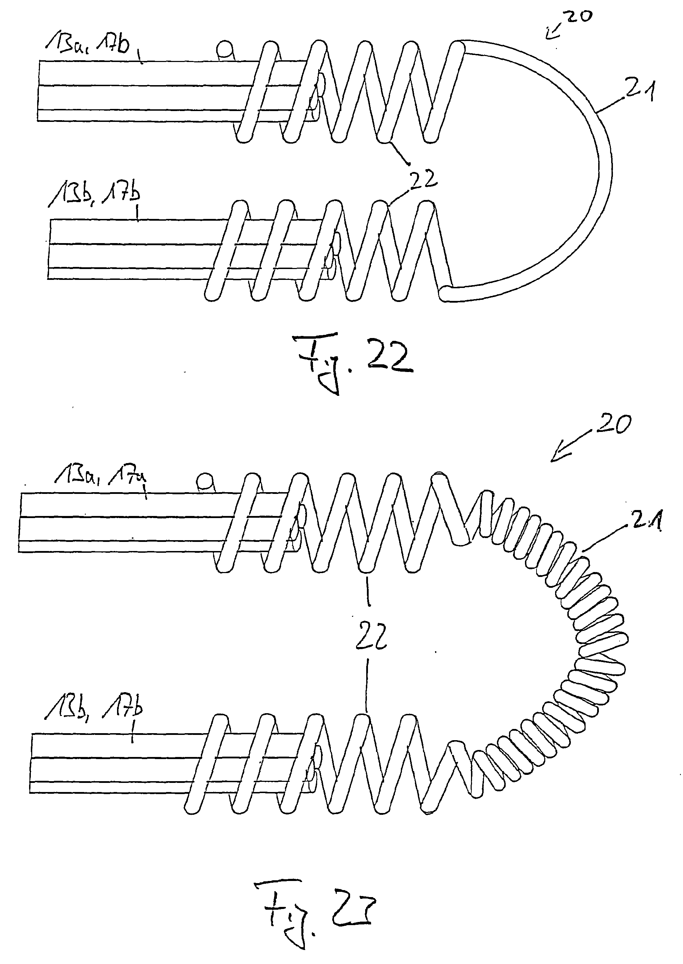

Bei dem Ausführungsbeispiel gemäß Fig. 22 umfasst das elastische Verbindungselement einen drahtförmigen mittleren Abschnitt 21, der vorgebogen sein kann. Der mittlere Abstand 21 ist einteilig mit den Verbindungsorganen 22, insbesondere den Coils, verbunden. An Stelle der Coils können andere Verbindungsorgane bzw. Verbindungsenden 22, beispielsweise Hülsen oder eine stoffschlüssige Verbindung, vorgesehen sein. Bei dem Ausführungsbeispiel gemäß Fig. 23 ist der mittlere Abschnitt 21 coilförmig ausgebildet. Der coilförmige mittlere Abschnitt 21 kann mit Coils verbunden sein, die die Verbindungsenden 22 bilden und einen größeren Durchmesser als der Coil des mittleren Abschnitts 21 aufweisen. Durch den kleineren Durchmesser des mittleren Coils lässt sich das Verbindungselement 20 leichter Crimpen. Beim Crimpen verkleinert sich der Biegeradius des Coils. Je kleiner das elastische Verbindungselement 20 ist, desto besser folgt das Element der Biegung. Dabei ist das Verhältnis zwischen Biegeradius und Querschnitt des elastischen Elements möglichst groß.

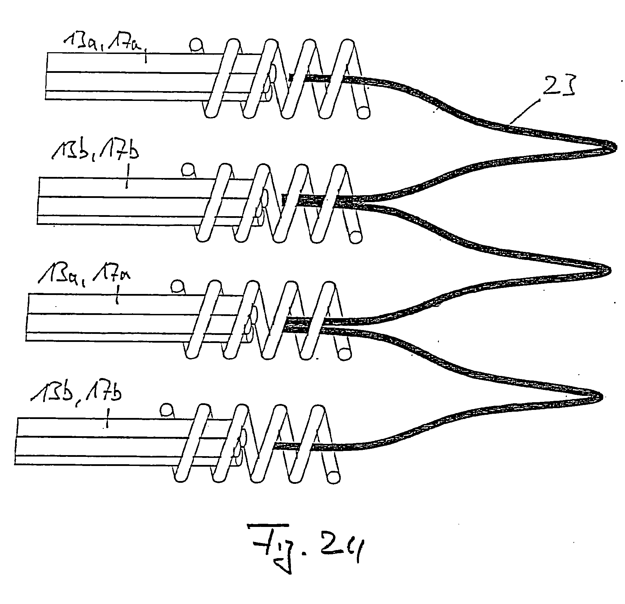

Wie in Fig. 24 dargestellt, kann das elastische Verbindungselement einen Mehrfachverbinder umfassen, der mehrere Geflechtenden 13a, 13b bzw. Verbindungsabschnitt 17a, 17b verbindet. Beispielsweise kann der Mehrfachverbinder ähnlich wie das Segment eines geschnittenen Stents ausgebildet sein.

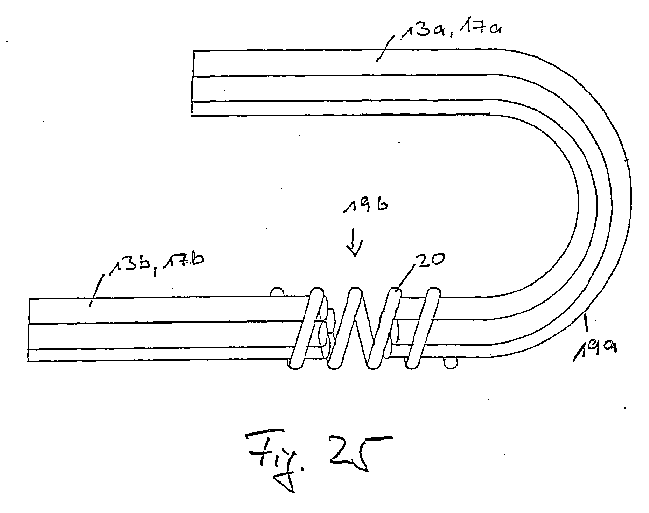

Ein besonders bevorzugtes Ausführungsbeispiel für die Verbindung der Geflechtenden 13a, 13b, 14a, 14b, 15a, 15b bzw. der Verbindungsabschnitte 17a, 17b ist in Fig. 25 dargestellt. Das Ausführungsbeispiel gemäß Fig. 25 beruht auf einem ähnlichen Prinzip wie die in den vorangegangenen Ausführungsbeispielen gezeigten Verbindungselemente 20, wobei die Rückstellkraft ebenfalls durch ein bogenförmiges Element erreicht wird, das den Abstand zwischen den beiden Geflechtenden 13a, 13b bzw. den Verbindungsabschnitten 17a, 17b überbrückt. Im Unterschied zu den vorangegangenen Ausführungsbeispielen wird hierfür jedoch kein separates Bauteil verwendet, sondern eines der beiden Geflechtenden 13a, 13b, das zu diesem Zweck gekrümmt ist. Der gekrümmte Bereich 19a des einen Geflechtendes 13a grenzt dabei an den Verbindungsbereich 19b zwischen den beiden verbundenen Geflechtenden 13a, 13b an. Die Verbindung wird durch ein elastisches oder auch nicht elastisches Verbindungselement 20, beispielsweise durch ein Coil oder andere Verbindungselemente, wie eine Hülse bewirkt, wobei das elastische Verbindungselement bevorzugt ist, da dieses auch in Biegung gehen kann. Auch hier sind stoff-, kraft- und reibschlüssige Verbindungen möglich. Der Verbindungsbereich 19b befindet sich außermittig bezogen auf die beiden Geflechtenden 13a, 13b, also nicht in der Mitte des Bügels bzw. des gekrümmten Bereichs 19a. Eine symmetrische Anordnung, bei der beide Geflechtenden 13a, 13b gekrümmt sind, ist denkbar. Es ist auch denkbar, das beide Geflechtenden 13a, 13b sich im Bogen überlappen und beide gebogen sind, wodurch sich die Kraft erhöht. Sie können dann auch durch Verbindungstechnik, wie Kleben, Schweißen und dgl. verbunden sein, ohne dass ein Verbindungselement erforderlich ist.

Das Ausführungsbeispiel gemäß Fig. 25 hat den Vorteil, das Geflechtende selbst in die Biegung geht, wodurch eine hohe Rückstellkraft mit einfachen Mitteln erreicht wird. Die Wirkung kann dadurch verbessert werden, dass die Verbindungsabschnitte 17a verdrillte oder geflochtene Drähte aufweisen. Insbesondere die Verdrillung der Drähte hat den Vorteil, dass sich kein Draht auf dem Außenrand der Biegung komplett befindet und dadurch übermäßig verformt wird. Durch das Ausführungsbeispiel gemäß Fig. 25 wird ein besonders gutes Zusammenspiel von Kraft und Flexibilität, wie bei einem Seil, erreicht.

Das Ausführungsbeispiel gemäß Fig. 25 ist auf alle Geflechtverbindungen anwendbar.

Das Geflecht, bzw. die Wandung 11 selbst kann aus Nitinol, oder aus CromCobalt Legierungen, aus Elgiloy Metall, oder aus Kunststoffdrähten hergestellt sein. Die Drähte sind bevorzugt gleich oder kleiner als 100 µm, 80 µm, 50 µm 40 µm. Es ist möglich, dass in einem oder mehreren Umfangsabschnitten zur besseren Verbindung ein Geflechtende gesplittet wird in 2 oder mehrere Geflechtenden, die dann im selben nächsten Geflechtende oder auch in unterschiedlichen Geflechtenden münden.

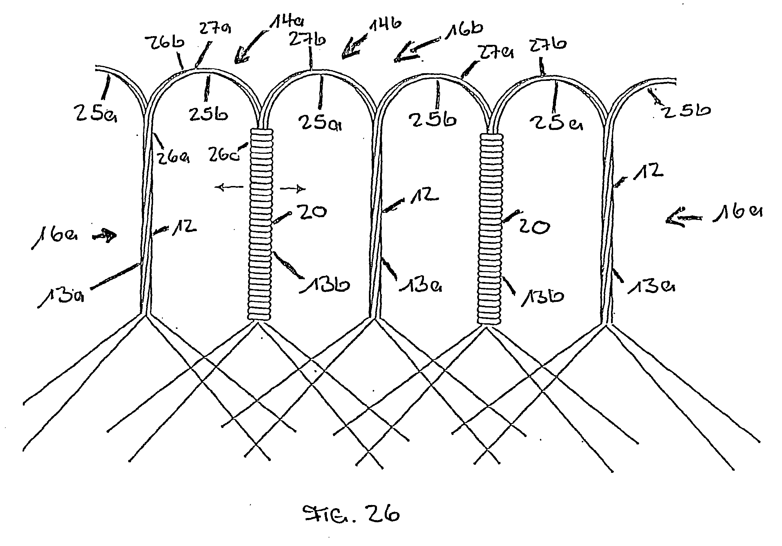

Weitere Ausführungsbeispiele für erfindungsgemäße Implantate, bei denen eine sich selbst stabilisierenden Schlaufenkonfiguration im Bereich der axialen Stentenden zum Einsatz kommt, sind in den Figuren 26 bis 30 gezeigt. Die Ausbildung der Stentaxialenden in Schlaufentechnik ist beispielsweise auf die in den Figuren 1 bis 8 dargestellten Ausführungsbeispiele, insofern, als das axiale Stentende abschließenden Geflechtenden, insbesondere die zweiten Geflechtenden durch die sich selbst stabilisierenden Schlaufentechnik erweitert werden. Die Begriffe "erste und zweite Geflechtenden" stehen allgemein für einander in Längsrichtung des Stents nachgeordnete Geflechtenden, die miteinander interagieren, ohne dabei auf Ausführungsbeispiele beschränkt zu sein, die nur zwei in Längsrichtung nachgeordnete Geflechtenden aufweisen, also nur zwei interagierende Ebenen.

Beim Ausführungsbeispiel gemäß Fig. 26 bedeutet dies, dass die Erfindung nicht auf die dargestellte Wechselwirkung der ersten Geflechtenden 13a, 13b, die den ersten Umfangsabschnitt 16a bilden, mit den zweiten Geflechtenden 14a, 14b, die den zweiten Umfangsabschnitt 16b bilden bzw. mit den das axiale Implantatende bildenden Geflechtenden eingeschränkt ist. Vielmehr ist es auch möglich, den in Fig. 26 dargestellten ersten Geflechtenden 16a weitere Geflechtenden in Längsrichtung vorzuordnen, wie beispielsweise in den Figuren 4 bis 6 dargestellt.

Aus Gründen der Einfachheit werden die das axiale Implantatende bildenden Geflechtenden nachfolgend als zweite Geflechtenden (im Sinne von auf die ersten Geflechtenden längsaxial nachfolgend angeordneten Geflechtenden) bezeichnet.

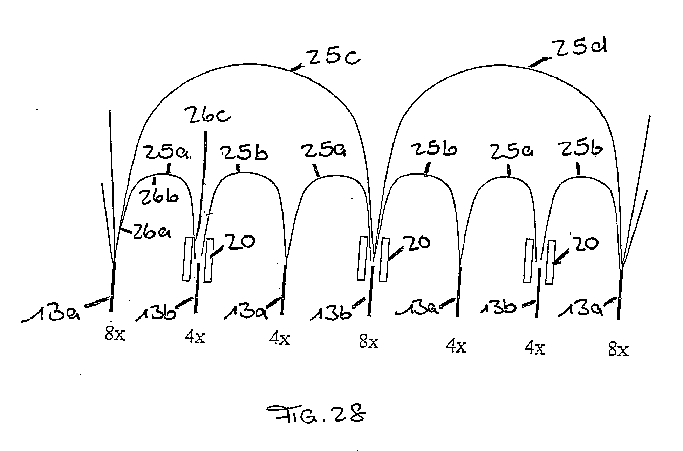

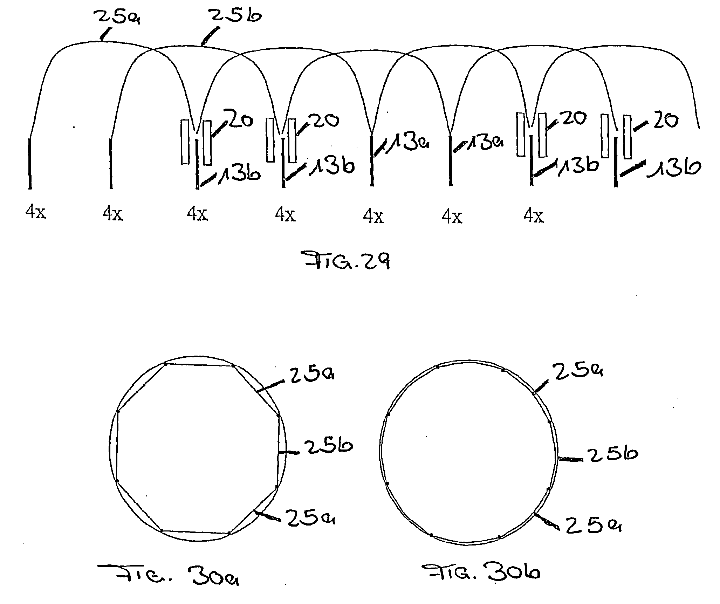

In Fig. 26 ist dargestellt, dass die zweiten Geflechtenden 14a, 14b als schlaufenförmige Enden 25a, 25b ausgebildet sind, die einen in distaler Richtung erstreckten ersten Abschnitt 26a, einen gekrümmten zweiten Abschnitt 26b und einen in proximaler Richtung erstreckten dritten Abschnitt 26c umfassen. Der gekrümmte zweite Abschnitt 26b ist zwischen den beiden in proximaler und distaler Richtung erstreckten ersten und dritten Abschnitten 26a, 26c angeordnet und verbindet diese. Basierend auf dieser Grundstruktur der Schlaufe bzw. der schlaufenförmigen Enden 25a, 25b ist die Geometrie der Schlaufe frei wählbar. Wie in Fig. 26 dargestellt, ist es besonders vorteilhaft, die ersten und dritten Abschnitte 26a, 26c in axialer Längsrichtung des Stents anzuordnen, wodurch eine Parallelanordnung der schlaufenförmigen Enden 25a, 25b und, damit verbunden, eine einfache und feste Anbindung der jeweiligen schlaufenförmigen Enden 25a, 25b mit benachbarten schlaufenförmigen Enden 25a, 25b erreicht wird. Die schlaufenförmigen Enden 25a, 25b verbinden im Wesentlichen sich axial erstreckende erste Geflechtenden 13a, 13b, die auf dem Umfang des Stents verteilt angeordnet sind. Dabei ist es möglich, dass die schlaufenförmigen Enden 25a, 25b, wie in Fig. 26 dargestellt, unmittelbar angrenzend angeordnete erste Geflechtenden 13a, 13b miteinander verbinden. Andere Konfigurationen, bei denen weiter entfernt angeordnete erste Geflechtenden miteinander verbunden sind, werden anhand der Figuren 28, 29 an anderer Stelle erläutert.

Die Gesamtstabilität des Geflechtes wird dadurch erhöht, dass wenigstens zwei in Umfangsrichtung unmittelbar angrenzend angeordnete schlaufenförmige Enden 25a, 25b miteinander verbunden sind. Bei dem Stent gemäß Fig. 26 sind alle in Umfangsrichtung unmittelbar angrenzend angeordnete schlaufenförmige Enden 25a, 25b miteinander verbunden. Es ist auch denkbar, eine andere Anzahl von schlaufenförmigen Enden 25a, 25b miteinander zu verbinden, wobei das beste Ergebnis für die Gesamtstabilität des Geflechtes erzielt wird, wenn alle schlaufenförmigen Enden, die unmittelbar benachbart angeordnet sind, miteinander verbunden sind.

Die Verbindung der einzelnen schlaufenförmigen Enden 25a, 25b miteinander erfolgt dadurch, dass die Schlaufenenden, konkret der erste und dritte Abschnitt 26a, 26c fixiert sind. Dies erfolgt bei dem Ausführungsbeispiel gemäß Fig. 26 beispielsweise dadurch, dass die sich distal erstreckenden ersten Abschnitte zweier in Umfangsrichtung unmittelbar benachbart angeordneter schlaufenförmiger Enden 25a, 25b aus dem selben ersten Geflechtende 13a kommend gebildet sind. Da das erste Geflechtende 13a eine Fixierung der darin enthaltenen Drahtenden 12 bewirkt, beispielsweise durch Verdrillen oder Verflechten der Drahtenden 12, sind die aus dem selben ersten Geflechtende 13a fortgeführten, insbesondere abgezweigten beiden ersten distalen Abschnitte 26a ebenfalls fixiert. Damit sind die in Umfangsrichtung benachbart angeordneten schlaufenförmigen Enden 25a, 25b in diesem Bereich miteinander verbunden derart, dass beim Verformen des Implantats bzw. des Stents praktisch keine Relativbewegung zwischen den angrenzenden Schlaufen 25a, 25b erfolgt.

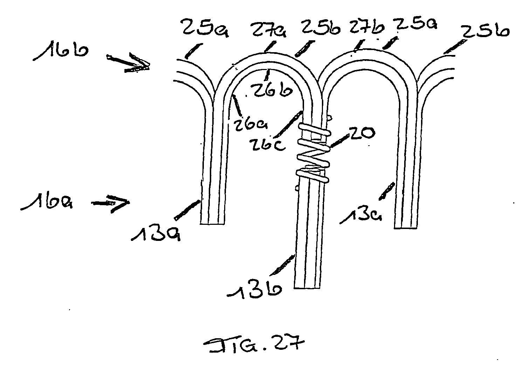

Dasselbe Prinzip liegt bei dem anderen Schlaufenende des schlaufenförmigen Endes 25a bzw. 25b vor. Dort sind die in proximaler Richtung erstreckten dritten Abschnitte 26c zweier in Umfangsrichtung unmittelbar benachbart angeordneter schlaufenförmiger Enden 25a, 25b zusammengeführt und zusammengefasst. Wie anhand Fig. 27 an anderer Stelle näher erläutert, sind die beiden zusammengeführten proximalen dritten Abschnitte 26c miteinander verbunden. Dies kann mechanisch durch Verdrillen, Verflechten und/oder durch Fixieren mittels eines Verbindungselementes 20 und/oder stoffschlüssig erfolgen. Durch die Verbindung der beiden proximalen dritten Abschnitte 26c werden die zugehörigen schlaufenförmigen Enden 25a, 25b fixiert und eine Relativbewegung zwischen den beiden schlaufenförmigen Enden 25a, 25b praktisch vermieden. Überdies sind die miteinander verbundenen proximalen dritten Abschnitte 26c mit einem dritten Drahtbündel verbunden, nämlich mit einem weiteren ersten Geflechtende 13b, das mit den beiden proximalen dritten Abschnitten 26c fluchtend angeordnet ist (Fig. 27).

Andere Arten der Verbindung angrenzender schlaufenförmiger Enden 25a, 25b sind möglich. Beispielsweise können die schlaufenförmigen Enden 25a, 25b als gesonderte Schlaufen hergestellt sein, die jeweils mit ersten Geflechtenden 13a, 13b durch Verbindungselemente 20 verbunden sind entsprechend der in Fig. 27 dargestellten mittleren Verbindung.

Ferner ist in Fig. 26 zu erkennen, dass nur ein Teil der Drahtenden 12, die jeweils ein erstes Geflechtende 13a, 13b, also ein in Axialrichtung sich erstreckendes Geflechtende bilden, zur Bildung der schlaufenförmigen Enden 25a, 25b herangezogen sind. Die schlaufenförmigen Enden 25a, 25b umfassen somit jeweils eine Gruppe 27a, 27b der Drahtenden 12 des ersten Geflechtendes 13a, 13b. Eine Gruppe 27a, 27b kann beispielsweise aus einem, mehr als einem, zwei, mehr als zwei, drei, mehr als drei, vier oder mehr als vier Drahtenden 12 bestehen. Die Gruppe 27a der Drahtenden 12 des ersten Geflechtendes 13a ist mit einer Gruppe 27b der Drahtenden 12 eines weiteren ersten Geflechtendes 13b verbunden, wobei die Gruppe 27b des weiteren ersten Geflechtendes 13b auf analoge Weise wie die Gruppe 27a des ersten Geflechtendes 13a gebildet ist.

Die Bildung der Gruppen 27a, 27b erfolgt bei dem Ausführungsbeispiel gemäß Fig. 26 so, dass die Drahtenden 12 des ersten Geflechts 13a im Bereich des zweiten Umfangsabschnittes 16b verzweigt sind. Das bedeutet, dass das erste Geflechtende 13a geteilt ist, wobei ein Teil der Drahtenden 12 eine Gruppe 27a und ein anderer Teil der Drahtenden 12 die weitere Gruppe 27b bildet. Wie in Fig. 26 weiter zu erkennen, bilden die beiden Gruppen 27a, 27b der Drahtenden 12 wenigstens zwei schlaufenförmige Enden 25a, 25b, die in entgegengesetzten Umfangsrichtungen angeordnet sind. Das sich so ergebende Schlaufengebilde hat eine schirmartige Form. Die aus einem gemeinsamen ersten Geflechtende 13a kommenden Drahtenden 12 sind durchgehend mit den Drahtenden 12 der verzweigten Gruppen 27a, 27b verbunden bzw. die Drahtenden 12 des gemeinsamen ersten Geflechtendes 13a bilden eine Fortführung die Drahtenden der beiden zugehörigen Gruppen 27a, 27b.

Die Drahtenden 12 des verzweigten ersten Geflechtendes 13a sind mit den Drahtenden 12 jeweils eines unverzweigten weiteren ersten Geflechtendes 13b verbunden, wobei das unverzweigte erste Geflechtende 13b im Bereich des ersten Umfangsabschnittes 16a, also proximal vom axialen Stentende, endet und zwar ohne Bildung von abgezweigten Drahtgruppen, die distal fortgeführt sind.

Wie in Fig. 26 zu erkennen, ist der in eine erste Umfangsrichtung geführte Drahtstrang bzw. die Gruppe 27a in proximaler Richtung unter Bildung des schlaufenförmigen Endes 25b in proximaler Richtung zurückgeführt und mit dem in der ersten Umfangsrichtung nächsten ersten Geflechtende 13b verbunden. Dieses erste Geflechtende 13 bildet ein unverzweigtes erstes Geflechtende 13b, wie anhand Fig. 27 an anderer Stelle näher erläutert. Der andere vom gemeinsamen ersten Geflechtende 13a kommende Drahtstrang bzw. die andere Gruppe 27b ist unter Bildung des schlaufenförmigen Endes 25a in proximaler Richtung zurückgeführt und in einer zweiten Umfangsrichtung, die zur ersten Umfangsrichtung entgegengesetzt ist, angeordnet. Die Gruppe 27b ist mit dem in der zweiten Umfangsrichtung nächsten ersten Geflechtende 13b verbunden, das wie an anderer Stelle anhand Fig. 27 erläutert, ein unverzweigtes Geflechtende bildet. Daraus ergibt sich ein schirmartiges Schlaufengebilde.

Die aus dem gemeinsamen ersten Geflechtende 13a kommenden verzweigten Gruppen 27a, 27b sind also jeweils mit in verschiedenen Umfangsrichtungen benachbart angeordneten ersten Geflechtenden 13b verbunden. Das bedeutet, dass sich die Schlaufenkonfiguration aus abwechselnd angeordneten verzweigten ersten Geflechtenden 13a und unverzweigten ersten Geflechtenden 13b zusammensetzt, die über den Umfang des Stents verteilt angeordnet sind.

Im Beispiel gemäß Fig. 26 sind die sich verzweigenden ersten Geflechtenden 13a aus jeweils vier Drahtenden 12 bzw. vier Drähten zusammengesetzt. Die verzweigten Drahtenden bzw. die Gruppen 27a, 27b bestehen jeweils aus zwei Drahtenden, die mit zwei weiteren Drahtenden 12 eines benachbarten sich verzweigenden weiteren ersten Geflechtendes 13a verbunden sind. Im Bereich der Verbindung der Gruppen 27a, 27b, die von verschiedenen gemeinsamen ersten Geflechtenden 13a kommen, sind also jeweils vier Drähte vorhanden, die miteinander verbunden sind.

Die Erfindung ist nicht auf die in Fig. 26 gezeigte Drahtanzahl beschränkt, sondern umfasst auch Drahtstränge bzw. Gruppen mit einer anderen Anzahl von Drähten. Beispielsweise können 2, 4, 6, 8, 10, 12, 14, 16 Drähte pro sich verzweigendem ersten Geflechtende 13a vorgesehen sein.

Die Verbindung der sich verzweigenden ersten Geflechtenden 13a mit den unverzweigten ersten Geflechtenden 13b ist in Fig. 27 dargestellt. Dort ist gut die Aufteilung der Drahtenden 12 in zwei Gruppen 26a, 27b zu erkennen, wobei jeweils zwei Gruppen von verschiedenen sich verzweigenden ersten Geflechtenden 13a zusammengeführt und miteinander verbunden sind. Die zusammengeführten Gruppen 27a, 27b sind mit einem unverzweigten ersten Geflechtende 13b verbunden. Dazu sind die die schlaufenförmigen Enden 25a, 25b bildenden Gruppen 27a, 27b in proximaler Richtung zurückgeführt derart, dass die Enden der Gruppen 27a, 27b mit dem zugehörigen unverzweigten ersten Geflechtende 13b fluchten.

Wie in Fig. 27 zu erkennen, ist die Verzweigung im Bereich des ersten distal sich erstreckenden Abschnittes 26a des schlaufenförmigen Endes 25a, 25b ausgebildet. Der erste distale Abschnitt 26a geht in den gekrümmten zweiten Abschnitt über, der wiederum in den sich proximal erstreckenden dritten Abschnitt 26c übergeht. Der dritte Abschnitt 26c bildet den Bereich, in dem zwei angrenzend angeordnete schlaufenförmige Enden 25a, 25b miteinander verbunden sind. Dabei sind die Drahtenden 12 des unverzweigten ersten Geflechtendes 13b und die Drahtenden 12 der zugehörigen schlaufenförmigen Enden 25a, 25b entgegengerichtet angeordnet. Konkret sind die Stirnflächen der miteinander verbundenen Geflechtenden gegenüber angeordnet derart, dass die Geflechtenden, d.h. die verzweigten und unverzweigten Geflechtenden 13a, 13b fluchten bzw. auf Stoß entgegengerichtet angeordnet sind. Im Bereich des proximalen dritten Abschnitts 26c sind die Drahtenden 12 der miteinander verbundenen Gruppen 27a, 27b parallel angeordnet.

Die Anordnung der Drahtenden 12 innerhalb einer Gruppe 27a, 27b bzw. im Bereich des ersten Geflechtendes 13a, 13b wird wie folgt erläutert.

Wie in Fig. 27 dargestellt, können die Drahtenden 12 im Bereich der schlaufenförmigen Enden 25a, 25b parallel angeordnet sein. Es ist auch möglich, die Drahtenden in diesen Bereichen zu verdrillen, zu verflechten oder anders zu verbinden, beispielsweise stoffschlüssig zu verbinden. Eine Kombination der vorstehend genannten Verbindungsarten ist insofern möglich, als die Drahtenden 12 beispielsweise im ersten distalen Abschnitt und im zweiten gekrümmten Abschnitt verflochten oder verdrillt und im dritten proximalen Abschnitt parallel angeordnet sind. Es ist auch möglich, die Drähte 12 der von den verschiedenen ersten Geflechtenden 13a kommenden Gruppen 27a, 27b miteinander zu verdrillen oder zu verflechten oder stoffschlüssig miteinander zu verbinden.

Zusätzlich ist zur Verbindung der von den verschiedenen ersten Geflechtenden 13a kommenden Gruppen 27a, 27b mit dem gegengerichtet angeordneten unverzweigten Geflechtende 13b ein Verbindungselement 20, beispielsweise in der Form eines Coils oder einer Hülse vorgesehen. Andere Ausbildungen des Verbindungselementes 20 sind möglich.

Das vorstehend genannte Flechten hat den Vorteil, dass die verschiedenen Abschnitte gut stabilisierbar sind. Demgegenüber ist das Verdrillen einfach realisierbar und mit sehr geringem Platzbedarf verbunden. Dadurch sind die Verbindungen durch kleine Verbindungselemente 20 sowie das Crimpen in kleine Durchmesser möglich. Wie vorstehend erwähnt, ist es möglich, dass das sich verzweigende erste Geflechtende 13a verflochten und die schlaufenförmigen Enden 25a, 25b verdrillt sind. Dadurch ist die Abzweigungsstelle zwischen dem sich verzweigenden ersten Geflechtendes 13a und den schlaufenförmigen Enden 25a, 25b gut stabilisiert wird. Es ist ferner möglich, dass der Bereich des unverzweigten ersten Geflechtendes 13b innerhalb des Verbindungselementes 20, beispielsweise innerhalb der Hülse verdrillt ist, so dass der Innendurchmesser des Verbindungselementes 20 klein gewählt werden kann. Der Bereich außerhalb der Hülse bzw. des Verbindungselementes 20 ist geflochten, so dass das Geflechtende stabil ist. Weitere Konfigurationen bzw. Kombinationen der unterschiedlichen Drahtverbindungsarten sind möglich.

Das beispielsweise als Verbindungselement 20 ausgebildete Coil oder die Hülse können mit den Drahtenden 12 verklebt, verschweißt und verlötet werden. Durch die stirnflächige Verbindung der schlaufenförmigen Enden 25a, 25b mit dem unverzweigten ersten Geflechtende wird Platz gespart, so dass das Verbindungselement 20, insbesondere der Coil bzw. die Hülse klein gewählt werden können.

Die Hülse kann mechanisch mit dem ersten Geflechtende bzw. den schlaufenförmigen Enden 25a, 25b verbunden sein, insbesondere durch Kaltverformung (Crimpen). Dabei ist die Hülse in die Zwischenräume der Drahtenden 12 eingeprägt bzw. bildet einen Hinterschnitt, so dass ein Formschluss zwischen der Hülse und den Drahtenden 12 entsteht. Diese Art der Verbindung ist sowohl im Bereich des ersten Geflechtendes 13a, 13b als auch im Bereich des sich proximal erstreckenden dritten Abschnittes 26c der schlaufenförmigen Enden 25a, 25b möglich.

Ein weiteres Ausführungsbeispiel für die selbststabilisierende Schlaufenkonfiguration ist in Fig. 28 dargestellt. Die vorstehend beschriebenen Verbindungsarten der verschiedenen Geflechtenden bzw. der Drahtenden 12 untereinander sind bei dem Ausführungsbeispiel gemäß Fig. 28 anwendbar. Dasselbe gilt für das Ausführungsbeispiel gemäß Fig. 29.

Die Grundstruktur der Schlaufenkonfiguration gemäß Fig. 26, bei der ein erstes Geflechtende 13a unter Bildung zweier in entgegengesetzten Umfangsrichtungen sich erstreckender schlaufenförmiger Enden 25a, 25b mit unverzweigten ersten Geflechtenden 13b verbunden sind, ist auch bei dem Ausführungsbeispiel gemäß Fig. 28 verwirklicht.

Die selbststabilisierende Wirkung des Schlaufengebildes wird dadurch erreicht, dass die schlaufenförmigen Enden 25a, 25b einerseits durch Aufteilung bzw. Verzweigung eines Drahtstranges bzw. ersten Geflechtendes gebildet wird, so dass die schlaufenförmigen Enden 25a, 25b, insbesondere deren erster distaler Abschnitt, durch die Verzweigung bzw. das der Verzweigung in Längsrichtung vorgeordnete Geflechtende verbunden sind. Andererseits sind die Enden, d.h. die proximal zurückgeführten dritten Abschnitte der schlaufenförmigen Enden 25a. 25b miteinander verbunden, so dass sich bereits daraus eine gewisse stabilisierende Wirkung ergibt. Die sich stabilisierende Wirkung wird dadurch verstärkt, dass die miteinander verbundenen proximal zurückgeführten dritten Abschnitte der schlaufenförmigen Enden 25a, 25b mit unverzweigten ersten Geflechtenden 13b verbunden sind, so dass die schlaufenförmigen Enden 25a, 25b auf beiden Seiten, d.h. sowohl im Bereich des ersten distal sich erstreckenden Abschnittes 26a als auch im Bereich des proximal zurückgeführten dritten Abschnitts 26c fixiert sind.

Zusätzlich zu der Ausgestaltung gemäß Fig. 26 ist bei dem Ausführungsbeispiel gemäß Fig. 28 vorgesehen, dass weitere schlaufenförmige Enden 25c, 25d vorgesehen sind, die mehrere erste Geflechtenden 13a, 13b übergreifen. Die Bildung der zusätzlichen schlaufenförmigen Enden 25c, 25d erfolgt analog zur Bildung der bereits erläuterten schlaufenförmigen Enden 25a, 25b, nämlich durch Verzweigung bzw. Aufteilung eines ersten Geflechtendes 13a einerseits und durch Verbindung des proximal zurückgeführten dritten Abschnittes des jeweiligen zusätzlichen schlaufenförmigen Endes 25c, 25d mit einem anderen proximalen dritten Abschnitt sowie einem unverzweigten ersten Geflechtende 13b. Dazu weisen die sich mehrfach verzweigenden ersten Geflechtenden 13c eine größere Anzahl von Drahtenden 12 auf, beispielsweise acht Drahtenden 12. Die acht Drahtenden werden in vier Gruppen mit je zwei Drahtenden 12 aufgeteilt, wobei zwei Gruppen die beiden schlaufenförmigen Enden 25a, 25b bilden, die die beiden in entgegengesetzten Umfangsrichtungen nächsten (unverzweigten) Geflechtenden 13b verbinden. Die beiden anderen schlaufenförmigen Enden 25d, 25c umfassen jeweils zwei Drahtenden 12, die mehrere erste Geflechtenden 13a, 13b überbrücken und im Bereich einer Mehrfachverbindungsstelle zusammengeführt sind. Das bedeutet, dass auf dem Umfang verteilt angeordnet mehrere erste Geflechtenden 13a, 13b angeordnet sind, die aus acht Drahtenden 12 gebildet sind, wobei zwischen den aus acht Drahtenden 12 gebildeten Geflechtenden jeweils zwei aus vier Drahtenden 12 gebildete Geflechtenden angeordnet sind. Eine andere Anzahl von Drahtenden pro mehrfachverzweigendem Geflechtende, beispielsweise 10, 12, 14 oder mehr als 14 sind möglich. Die Anzahl der zwischen den mehrfachverzweigenden bzw. mehrfachverbindenden Geflechtenden 13a, 13b angeordneten Geflechtenden 13a, 13b weisen eine geringere Zahl von Drahtenden 12 auf, insbesondere die Hälfte der Anzahl der Drahtenden 12 der mehrfachverzweigenden bzw. mehrfachverbindenden Geflechtenden 13a, 13 b.

Die Anzahl der übergriffenen ersten Geflechtenden 13a, 13b kann nach Bedarf variiert werden. Außerdem ist es möglich, mehr als die in Fig. 28 gezeigten zwei Ebenen vorzusehen, wobei das in Fig. 28 dargestellte Prinzip entsprechend erweitert bzw. fortgeführt wird.

Bei dem Ausführungsbeispiel gemäß Fig. 29 sind die schlaufenförmigen Enden 25a, 25b überlappend angeordnet. Dies wird dadurch erreicht, dass die schlaufenförmigen Enden 25a bzw. 25b jeweils ein erstes Geflechtende 13b übergreifen, so dass ein schlaufenförmiges Ende 25a jedes zweite Geflechtende 13b verbindet. Dabei sind jeweils zwei sich verzweigende erste Geflechtenden 13a in Umfangsrichtung nebeneinander angrenzend angeordnet. Dasselbe gilt für die verbindenden ersten Geflechtenden 13b, die ebenfalls jeweils paarweise bzw. allgemein jeweils gruppiert angeordnet sind.

Auch hier gilt, dass die Anzahl der Drahtenden 12 pro Geflechtende 13a, 13b variiert werden kann.

Bei den vorstehend beschriebenen Ausführungsbeispielen ist der zweite Umfangsabschnitt 16b durch die schlaufenförmigen Enden 25a, 25b gebildet, die den zweiten Geflechtenden 14a, 14b entsprechen. Der erste Umfangabschnitt 16a ist durch die ersten Geflechtenden 13a, 13b gebildet, die die schlaufenförmigen Enden 25a, 25b fixieren derart, dass die schlaufenförmigen Enden 25a, 25b zumindest im Übergangsbereich zwischen dem ersten und zweiten Umfangsabschnitt 16a, 16b miteinander verbunden sind. Bei den Ausführungsbeispielen gemäß den Figuren 26 bis 30 bildet der zweite Umfangsabschnitt 16b einen atraumatischen Endabschnitt, der das axiale Ende des Implantats bzw. des Stents begrenzt. Der Verbindungsbereich bzw. der punktuelle Verbindungsbereich zwischen den schlaufenförmigen Enden 25a, 25b und den ersten Geflechtenden 13a, 13b kann als Übergangsbereich zwischen dem ersten und zweiten Umfangsabschnitt angesehen werden. Wie in Fig. 26 zu erkennen, ist der zweite Umfangsabschnitt 16b dem ersten Umfangsabschnitt 16a insofern in längsaxialer Richtung nachgeordnet, als zumindest der gekrümmte zweite Abschnitt 26b der schlaufenförmigen Enden 25a, 25b dem sich in axialer Richtung erstreckenden ersten Geflechtenden 13a nachgeordnet ist.

In den Figuren 30a, 30b ist das Verhalten der schlaufenstabilisierten Stentenden im Gefäß dargestellt. Der Vorteil der schlaufenförmigen Enden 25a, 25b besteht darin, dass diese ein zylindrisches Profil aufweisen, so dass sie an der Gefäßwand anliegen, ohne in das Lumen hineinzuragen.

Bei der Herstellung ergeben sich aus der Verbindung der Geflechtenden, die in Fig. 30a durch Punkte markiert sind, gerade Schlaufen. Durch eine Verformung der Schlaufen bei der Herstellung nehmen diese ein zylindrisches Profil an, wie in Fig. 30b dargestellt. Die Verformung der schlaufenförmigen Enden 25a, 25b erfolgt bei der Herstellung derart, dass diese den Radius des Stents bzw. des Zielgefäßes annehmen. Dies kann beispielsweise dadurch erfolgen, dass die Drähte aus einem Formgedächtniswerkstoff, beispielsweise aus Nitinol hergestellt sind, wobei die schlaufenförmigen Enden 25a, 25b auf einem Dorn gehalten und dann wärmebehandelt werden, so dass eine entsprechende Konditionierung vorgenommen wird.

Die Funktion bzw. Wirkung der sich gegenseitig stabilisierenden Schlaufen bzw. schlaufenförmigen Enden 25a, 25b wird durch das Kräftegleichgewicht im Bereich des Stentendes erreicht. Das Kräftegleichgewicht ergibt sich aus der Symmetrie der Anordnung, bei der die einzelnen schlaufenförmigen Enden 25a, 25b miteinander verbunden sind und sich so gegenseitig fixieren.

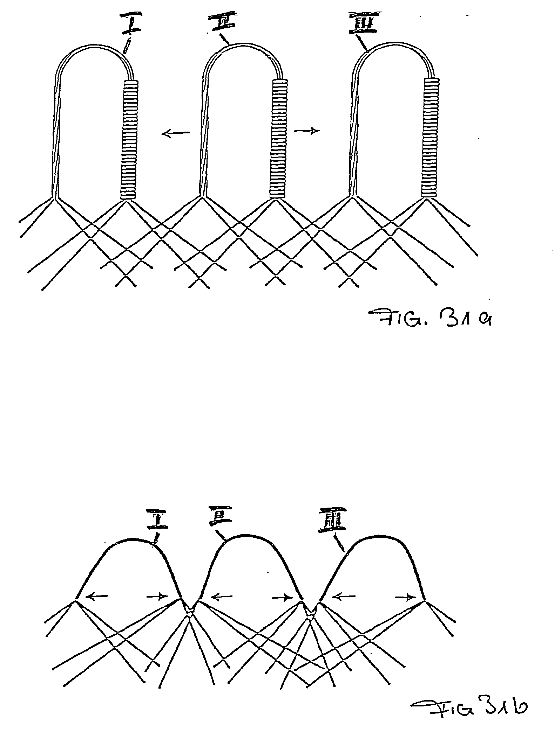

Im Unterschied dazu kommt es bei nichtstabilisierten Schlaufengebilden, wie beispielsweise in den Figuren 31a bis 31d dargestellt, durch die Rückstellkraft der einzelnen Schlaufe zu einer Relativbewegung der jeweiligen Geflechtenden, wobei die jeweiligen Geflechtenden auseinandergedrückt werden. Dies hat zur Folge, dass sich zwei Enden benachbarter Schlaufen einander annähern (Fig. 31b). Die Kraft der Schlaufen, die zur Verschiebung der Enden führt, trägt nicht zur Erweiterung des Durchmessers des Stents bei. Im Gegensatz dazu entfernen sich bei dm Ausführungsbeispielen gemäß den Figuren 26 bis 30 alle Geflechtenden voneinander, was zur Erweiterung des Stentdurchmessers auch im Bereich der Axialenden führt.

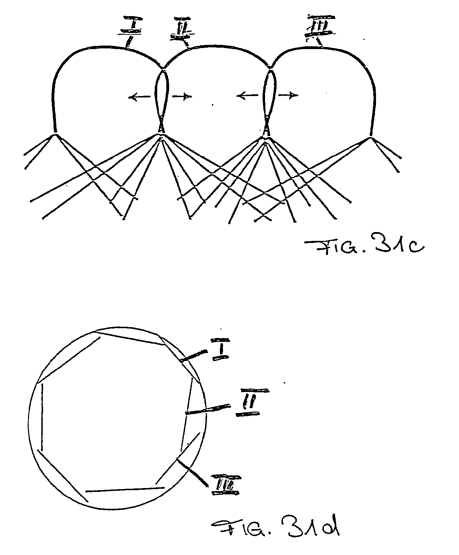

Das den Ausführungsbeispielen gemäß den Figuren 26 bis 30 zugrundeliegende Prinzip verhindert eine Überlappung der Schlaufen, weil sich bei den Ausführungsbeispielen gemäß Figuren 26 bis 30 zwei Nachbarschlaufen ein gemeinsames Ende teilen. In diesem Zusammenhang erfolgt die Überlappung ungewollt (Fig. 31c) und entspricht nicht der gezielt eingestellten Überlappung der schlaufenförmigen Enden 25a, 25b gemäß Fig. 29. Durch die schlaufenstabilisierten Geflechtenden wird verhindert, dass die Schlaufen im Gefäßlumen nach innen hinausragen, wie dies bei einem nichtstabilisierten Gebilde (Fig. 31d) der Fall ist.

### Bezugszeichenliste

- 10a, 10b, 10c, 10d: Drähte
- 11: Wandung
- 12a, 12b: Drahtenden
- 13a, 13b: erste Geflechtenden
- 14a, 14b: zweite Geflechtenden
- 15a, 15b: weitere Geflechtenden
- 16a: erster Umfangsabschnitt
- 16b: zweiter Umfangsabschnitt
- 16c: weitere Umfangsabschnitte
- 17a, 17b: Verbindungsabschnitte
- 18a, 18b: Stirnflächen
- 19a: gekrümmter Bereich
- 19b: Verbindungsbereich
- 20: Verbindungselement
- 21: mittlerer Abschnitt
- 22: Verbindungsenden
- 23: Mehrfachverbinder
- 24: Überkreuzungspunkte
- 25a, 25b,25c, 25d: schlaufenförmige Enden
- 26a, 26b, 26c: 1., 2., 3. Abschnitt
- 27a, 27b: Gruppen

## Patentansprüche

1. Medizinisches Implantat, insbesondere Stent, mit einer aus mehreren Drähten (10a, 10b, 10c, 10d) geflochtenen Wandung (11), die sich entlang einer Längsachse L erstreckt und zumindest abschnittsweise um die Längsachse L gekrümmt ist, wobei jeweils wenigstens zwei Drahtenden (12) der Drähte (10a, 10b, 10c, 10d) zu wenigstens zwei ersten Geflechtenden (13a, 13b) verbunden sind, die einen um die Längsachse L erstreckten ersten Umfangsabschnitt (16a) der Wandung (11) bilden,
**dadurch gekennzeichnet, dass**
jeweils wenigstens zwei erste Geflechtenden (13a, 13b) zu einem oder mehreren zweiten Geflechtenden (14a, 14b) verbunden sind, wobei die zweiten Geflechtenden (14a, 14b) einen um die Längsachse L erstreckten zweiten Umfangsabschnitt (16b) der Wandung (11) bilden, der dem ersten Umfangsabschnitt (16a) in längsaxialer Richtung nachgeordnet ist oder die zweiten Geflechtenden (14a, 14b) in Umfangsrichtung U der Wandung (11) angeordnet sind.

2. Implantat nach Anspruch 1,
**dadurch gekennzeichnet, dass**
die das axiale Implantatende, insbesondere das axiale Stentende, bildenden zweiten Geflechtenden (14a, 14b) schlaufenförmige Enden (25a, 25b, 25c, 25d) mit einem in distaler Richtung erstreckten ersten Abschnitt (26a), einem gekrümmten zweiten Abschnitt (26b) und einem in proximaler Richtung erstreckten dritten Abschnitt (26c) umfassen, die in Umfangsrichtung verteilt, im Wesentlichen sich axial erstreckende, erste Geflechtenden (13a, 13b) verbinden, wobei wenigstens zwei in Umfangsrichtung unmittelbar angrenzend angeordnete schlaufenförmige Enden (25a, 25b) miteinander verbunden sind.

3. Implantat nach Anspruch 2,
**dadurch gekennzeichnet, dass**
die schlaufenförmigen Enden (25a, 25b, 25c, 25d) jeweils eine Gruppe (27a) der Drahtenden (12) des ersten Geflechtendes (13a, 13b) umfassen, die mit einer Gruppe (27b) der Drahtenden (12) eines weiteren ersten Geflechtendes (13a, 13b) verbunden sind.

4. Implantat nach Anspruch 3,
**dadurch gekennzeichnet, dass**
die Drahtenden (12) des ersten Geflechtendes (13a, 13b) im Bereich des zweiten Umfangsabschnitts (16b) verzweigt sind derart, dass die Gruppen (27a, 27b) der Drahtenden (12) wenigstens zwei schlaufenförmige Enden (25a, 25b, 25c, 25d) bilden, die in entgegengesetzten Umfangsrichtungen angeordnet sind, wobei die Drahtenden (12) des einen verzweigten ersten Geflechtendes (13a, 13b) mit den Drahtenden (12) jeweils eines weiteren unverzweigten ersten Geflechtendes (13a, 13b) verbunden sind.

5. Implantat nach Anspruch 4,
**dadurch gekennzeichnet, dass**
die Drahtenden (12) des unverzweigten ersten Geflechtendes (13a, 13b) und die Drahtenden (12) der mit diesem verbundenen schlaufenförmigen Enden (25a, 25b, 25c, 25d) entgegengerichtet, insbesondere auf Stoß entgegengerichtet angeordnet sind.

6. Implantat nach wenigstens einem der Ansprüche 3 bis 5,
**dadurch gekennzeichnet, dass**
die Drahtenden (12) der untereinander verbundenen Gruppen (27a, 27b) im Bereich der Verbindung parallel angeordnet sind.

7. Implantat nach wenigstens einem der Ansprüche 2 bis 6,
**dadurch gekennzeichnet, dass**
die Drahtenden (12) des ersten Geflechtendes (13a, 13b) untereinander und/oder mit Drahtenden (12) wenigstens eines weiteren ersten Geflechtendes (13a, 13b) mechanisch und/oder stoffschlüssig verbunden sind.

8. Implantat nach wenigstens einem der Ansprüche 2 bis 7,
**dadurch gekennzeichnet, dass**
die schlaufenförmigen Enden (25a, 25b, 25c, 25d) mehrere Ebenen (28a, 28b) bilden derart, dass wenigstens ein schlaufenförmiges Ende (25a, 25b, 25c, 25d) mehrere erste Geflechtenden (13a, 13b) übergreift, die durch schlaufenförmige Enden (25a, 25b, 25c, 25d) miteinander verbunden sind.

9. Implantat nach wenigstens einem der Ansprüche 2 bis 8,
**dadurch gekennzeichnet, dass**
die schlaufenförmigen Enden (25a, 25b, 25c, 25d) überlappend angeordnet sind.

10. Implantat nach wenigstens einem der Ansprüche 1 bis 9,
**dadurch gekennzeichnet, dass**
die Drähte (10a, 10b) im Bereich nahe der Verbindung der Drahtenden (12) und die ersten Geflechtenden (13a, 13b) unterschiedliche Flechtwinkel aufweisen und/oder die in axial nachgeordneten Umfangsabschnitten (16a, 16b, 16c) vorgesehenen Geflechtenden (13a, 13b, 14a, 14b, 15a, 15b) unterschiedliche Flechtwinkel aufweisen.

11. Implantat nach wenigstens einem der Ansprüche 1 bis 10,
**dadurch gekennzeichne**t, dass
zwei miteinander verbundene Geflechtenden (13a, 13b, 14a, 14b, 15a, 15b) wenigstens ein Geflechtende aufweisen, das angrenzend an den Verbindungsbereich (19b) zwischen den beiden verbundene Geflechtenden (13a, 13b, 14a, 14b, 15a, 15b) gekrümmt ist.

12. Implantat nach Anspruch 11,
**dadurch gekennzeichnet, dass**
die Drähte (10a, 10b, 10c, 10d) des gekrümmten Geflechtendes verdrillt oder gewickelt sind.

13. Verfahren zur Herstellung eines medizinischen Implantats nach einem der vorhergehenden Ansprüche, bei dem eine Wandung (11) aus mehreren Drähten (10a, 10b, 10c, 10d) geflochten wird, wobei jeweils zwei Drahtenden (12) der Drähte (10a, 10b, 10c, 10d) miteinander zur Bildung wenigstens zweier Geflechtenden (13a, 13b) verbunden werden, und wobei
wenigstens zwei Geflechtenden (13a, 13b) miteinander verbunden werden.

## Claims

1. A medical implant, in particular a stent, having a wall (11) braided from a plurality of wires (10a, 10b, 10c, 10d) which extends along a longitudinal axis L and is curved about the longitudinal axis L at least in sections, wherein respectively, at least two wire ends (12) of the wires (10a, 10b, 10c, 10d) are connected to at least two first braid ends (13a, 13b) which form a first circumferential section (16a) of the wall (11) extending about the longitudinal axis L,
**characterized in that**
respectively, at least two first braid ends (13a, 13b) are connected to one or more second braid ends (14a, 14b), wherein the second braid ends (14a, 14b) form a second circumferential section (16b) of the wall (11) which extends about the longitudinal axis L which is axially subordinate to the first circumferential section (16a) in the longitudinal axial direction, or the second braid ends (14a, 14b) are disposed in the circumferential direction U of the wall (11).

2. The implant as claimed in claim 1,
**characterized in that**
the second braid ends (14a, 14b) forming the axial implant end, in particular the axial stent end, comprise loop-shaped ends (25a, 25b, 25c, 25d) with a first section (26a) extending in the distal direction, a curved second section (26b) and a third section (26c) extending in the proximal direction which connect the first braid ends (13a, 13b) which are distributed in the circumferential direction and extend essentially axially, wherein at least two loop-shaped ends (25a, 25b) which are immediately adjacently disposed in the circumferential direction are connected together.

3. The implant as claimed in claim 2,
**characterized in that**
the loop-shaped ends (25a, 25b, 25c, 25d) respectively comprise a group (27a) of the wire ends (12) of the first braid end (13a, 13b) which are connected with a group (27b) of the wire ends (12) of a further first braid end (13a, 13b).

4. The implant as claimed in claim 3,
**characterized in that**
the wire ends (12) of the first braid end (13a, 13b) are branched in the region of the second circumferential section (16b) in a manner such that the groups (27a, 27b) of the wire ends (12) form at least two loop-shaped ends (25a, 25b, 25c, 25d) which are disposed in opposite circumferential directions, wherein the wire ends (12) of the one branched first braid end (13a, 13b) are connected with the wire ends (12) of respectively a further non-branched first braid end (13a, 13b).

5. The implant as claimed in claim 4,
**characterized in that**
the wire ends (12) of the non-branched first braid end (13a, 13b) and the wire ends (12) of the loop-shaped ends (25a, 25b, 25c, 25d) connected thereto are disposed in the opposite direction, and in particular are abutted against each other.

6. The implant as claimed in at least one of claims 3 to 5,
**characterized in that**
the wire ends (12) of the mutually connected groups (27a, 27b) are disposed in a parallel manner in the region of the connection.

7. The implant as claimed in at least one of claims 2 to 6,
**characterized in that**
the wire ends (12) of the first braid end (13a, 13b) are mechanically connected and/or materially bonded among themselves and/or with wire ends (12) of at least one further first braid end (13a, 13b).

8. The implant as claimed in at least one of claims 2 to 7,
**characterized in that**
the loop-shaped ends (25a, 25b, 25c, 25d) form a plurality of planes (28a, 28b) in a manner such that at least one loop-shaped end (25a, 25b, 25c, 25d) spans a plurality of first braid ends (13a, 13b) which are connected together by means of loop-shaped ends (25a, 25b, 25c, 25d).

9. The implant as claimed in at least one of claims 2 to 8,
**characterized in that**
the loop-shaped ends (25a, 25b, 25c, 25d) are disposed in an overlapping manner.

10. The implant as claimed in at least one of claims 1 to 9,
**characterized in that**
in the region close to the connection of the wire ends (12) and the first braid ends (13a, 13b), the wires (10a, 10b) have different braid angles and/or the braid ends (13a, 13b, 14a, 14b, 15a, 15b) provided in axially subordinate circumferential sections (16a, 16b, 16c) have different braid angles.

11. The implant as claimed in at least one of claims 1 to 10,
**characterized in that**
two mutually connected braid ends (13a, 13b, 14a, 14b, 15a, 15b) comprise at least one braid end which is curved adjacent to the connection region (19b) between the two connected braid ends (13a, 13b, 14a, 14b, 15a, 15b).

12. The implant as claimed in claim 11,
**characterized in that**
the wires (10a, 10b, 10c, 10d) of the curved braid end are twisted or coiled.

13. A method for the manufacture of a medical implant as claimed in one of the preceding claims, in which a wall (11) is braided from a plurality of wires (10a, 10b, 10c, 10d), wherein respectively two wire ends (12) of the wires (10a, 10b, 10c, 10d) are connected together to form at least two braid ends (13a, 13b), and wherein
at least two braid ends (13a, 13b) are connected together.

## Revendications

1. Implant médical, en particulier stent, comprenant une paroi (11) de plusieurs fils (10a, 10b, 10c, 10d) en treillis qui s'étend le long d'un axe longitudinal L et est courbée au moins par tronçons le long de l'axe longitudinal L, dans lequel respectivement au moins deux extrémités de fil (12) des fils (10a, 10b, 10c, 10d) sont reliées à au moins deux premières extrémités de treillis (13a, 13b) qui forment un premier tronçon périphérique (16a) de la paroi (11) s'étendant autour de l'axe longitudinal L,
**caractérisé en ce que**
respectivement au moins deux premières extrémités de treillis (13a, 13b) sont reliées à une ou plusieurs deuxièmes extrémités de treillis (14a, 14b), dans lequel les deuxièmes extrémités de treillis (14a, 14b) forment un deuxième tronçon périphérique (16b) de la paroi (11) s'étendant autour de l'axe longitudinal L, qui est placé après le premier tronçon périphérique (16a) dans le sens axial longitudinal ou bien les deuxièmes extrémités de treillis (14a, 14b) sont disposées dans le sens périphérique U de la paroi (11).

2. Implant selon la revendication 1,
**caractérisé en ce que**
les deuxièmes extrémités de treillis (14a, 14b) formant l'extrémité d'implant axiale, en particulier l'extrémité de stent axiale, comprennent des extrémités en forme de passant (25a, 25b, 25c, 25d) avec un premier tronçon (26a) s'étendant dans le sens distal, un deuxième tronçon courbé (26b) et un troisième tronçon (26c) s'étendant dans le sens proximal qui, répartis sur le sens périphérique, relient essentiellement des premières extrémités de treillis (13a, 13b) s'étendant dans le sens axial, dans lequel au moins deux extrémités en forme de passant (25a, 25b) disposées directement adjacentes dans le sens périphérique, sont reliées entre elles.

3. Implant selon la revendication 2,
**caractérisé en ce que**
les extrémités en forme de passant (25a, 25b, 25c, 25d) comprennent respectivement un groupe (27a) des extrémités de fil (12) de la première extrémité de treillis (13a, 13b) qui sont reliées à un groupe (27b) des extrémités de fil (12) d'une autre première extrémité de treillis (13a, 13b).

4. Implant selon la revendication 3,
**caractérisé en ce que**
les extrémités de fil (12) de la première extrémité de treillis (13a, 13b) sont ramifiées de telle façon au niveau du deuxième tronçon périphérique (16b), que les groupes (27a, 27b) des extrémités de fil (12) forment au moins deux extrémités en forme de passant (25a, 25b, 25c, 25d) qui sont disposées dans des sens périphériques opposés, dans lequel les extrémités de fil (12) de l'une première extrémité de treillis (13a, 13b) ramifiée sont reliées aux extrémités de fil (12) respectivement d'une autre extrémité de treillis (13a, 13b) non ramifiée.

5. Implant selon la revendication 4,
**caractérisé en ce que**
les extrémités de fil (12) de la première extrémité de treillis (13a, 13b) non ramifiée et les extrémités de fil (12) des extrémités en forme de passant (25a, 25b, 25c, 25d) reliées à celle-ci sont disposées dirigées en sens opposé, en particulier dirigées en sens opposé bord à bord.

6. Implant selon au moins l'une des revendications 3 à 5,
**caractérisé en ce que**
les extrémités de fil (12) des groupes (27a, 27b) reliés entre eux, sont disposées parallèles au niveau de la liaison.

7. Implant selon au moins l'une des revendications 2 à 6,
**caractérisé en ce que**
les extrémités de fil (12) de la première extrémité de treillis (13a, 13b) sont reliées mécaniquement et/ou par complémentarité de matière entre elles et/ou aux extrémités de fil (12) d'au moins une autre première extrémité de treillis (13a, 13b).

8. Implant selon au moins l'une des revendications 2 à 7,
**caractérisé en ce que**
les extrémités en forme de passant (25a, 25b, 25c, 25d) forment plusieurs plans (28a, 28b) de telle façon qu'au moins une extrémité en forme de passant (25a, 25b, 25c, 25d) englobe plusieurs premières extrémités de treillis (13a, 13b) qui sont reliées entre elles par des extrémités en forme de passant (25a, 25b, 25c, 25d).

9. Implant selon au moins l'une des revendications 2 à 8,
**caractérisé en ce que**
les extrémités en forme de passant (25a, 25b, 25c, 25d) sont disposées en se chevauchant.

10. Implant selon au moins l'une des revendications 1 à 9,
**caractérisé en ce que**
les fils (10a, 10b), à proximité de la liaison des extrémités de fil (12), ainsi que les première extrémités de treillis (13a, 13b), présentent différents angles de treillis, et/ou les extrémités de treillis (13a, 13b, 14a, 14b, 15a, 15b) prévues dans des tronçons périphériques (16a, 16b, 16c) montés à la suite axialement présentent différents angles de treillis.

11. Implant selon au moins l'une des revendications 1 à 10,
**caractérisé en ce que**
deux extrémités de treillis (13a, 13b, 14a, 14b, 15a, 15b) reliées entre elles présentent au moins une extrémité de treillis qui est coudée entre les deux extrémités de treillis reliées (13a, 13b, 14a, 14b, 15a, 15b) dans le voisinage de la zone de liaison (19b).

12. Implant selon la revendication 11,
**caractérisé en ce que**
les fils (10a, 10b, 10c, 10d) de l'extrémité de treillis coudée sont torsadés ou enroulés.

13. Procédé de fabrication d'un implant médical selon l'une des revendications précédentes, dans lequel une paroi (11) est constituée d'un treillis de plusieurs fils (10a, 10b, 10c, 10d), dans lequel respectivement deux extrémités de fil (12) des fils (10a, 10b, 10c, 10d) sont reliées entre elles pour former au moins deux extrémités de treillis (13a, 13b), et dans lequel
au moins deux extrémités de treillis (13a, 13b) sont reliées entre elles.
